# EUROPEAN PATENT SPECIFICATION

(11) **EP 4 037 600 B1**
(45) Date of publication and mention of the grant of the patent: **02.12.2026**
(21) Application number: 20871139.0
(22) Date of filing: 02.10.2020
(51) Int. Cl.: A61B 50/30, A61B 46/10, A61B 17/16

(54) **DISPOSABLE STERILE COVER SYSTEM, COMPONENTS, AND METHODS FOR POWER TOOLS**
STERILES EINWEG-ABDECKSYSTEM, KOMPONENTEN UND VERFAHREN FÜR ELEKTROWERKZEUGE
SYSTÈME DE PROTECTION STÉRILE À USAGE UNIQUE, COMPOSANTS ET PROCÉDÉS POUR OUTILS ÉLECTRIQUES

(30) Priority: 02.10.2019 US 201962909441 P
(43) Date of publication of application: 10.08.2022
(73) Proprietor: Arbutus Medical Inc., Vancouver, BC V5Y 3E9 (CA)
(72) Inventor: CANCILLA, Michael, Vancouver, British Columbia V5P 1A2 (CA); MULABDIC, Fedja, Vancouver, British Columbia V7P 3G8 (CA); BHATLA, Chris, Vancouver, British Columbia V6E 1E2 (CA)
(74) Representative: Bardehle Pagenberg Partnerschaft mbB Patentanwälte Rechtsanwälte
(86) International application number: PCT/CA2020/051318
(87) International publication number: WO 2021/062548

(56) References cited:
- WO-A1-97/32534
- JP-B2- 4 570 294
- US-A1- 2014 262 408
- US-A1- 2016 242 861
- US-A1- 2017 027 658
- US-A1- 2017 325 791
- US-B1- 6 716 215
- US-B2- 8 944 069

## Description

### Cross-Reference to Related Application

The present application claims priority to United States Provisional Application No. 62/909,441, filed on October 2, 2019, entitled "DISPOSABLE STERILE COVER SYSTEM FOR POWER TOOLS".

### Field

The technology described herein relates generally to covers for power tools. The power tools have example application in surgery. Embodiments of the present technology include a novel disposable sterile cover, cover system, components, and methods related to providing a barrier between a non-sterile power tool and a sterile field.

### Background

Power tools such as drills and saws are used in various surgical procedures, such as for bone stabilization, to repair a fracture, in joint replacement procedures, in joint reconstruction procedures, and for bone removal (osteotomy). A drill may also, for example, be used by an orthopedic surgeon to drill a hole in bone to receive a screw or wire. Since surgery must be performed with sterile equipment, surgical tools such as surgical drills are designed to survive sterilization procedures. Such surgical tools can be exceedingly expensive. Specialized surgical tools can be so expensive that they may be unaffordable and may reduce access to surgical care. In regions of the world where surgery is readily available, expensive surgical tools may contribute to the rising cost of healthcare.

Battery powered portable tools for use by tradespeople and homeowners are commonly available and are much less expensive than specialized surgical tools. Research has shown that these commonly available tools could meet performance requirements required for surgery and satisfy requirements such as speed, torque, weight, ergonomics, electrical safety and the like. However, conventional power tools cannot be effectively sterilized and are therefore not suitable on their own for use in sterile procedures.

Some communities rely on surgical procedures performed by a visiting team of medical professionals who travel with their equipment. Additionally, military surgeons, for example, require portability when deploying surgical teams near or in conflict zones. The possibility of using portable power tools instead of specialized surgical tools would be advantageous in both of these example scenarios.

United States Patent No. 10,405,937 describes power tool covers that may withstand multiple sterilization cycles. Such covers are reusable and have been shown to be very useful.

United States Patent Application Publication No. US 2017/0325791 A1 discloses a device for sterile coupling of a percutaneous surgical instrument to a rotary drive tool. The device has a flexible envelope intended to confine the drive tool, a connector piece for coupling to the percutaneous surgical instrument via one end and to the drive tool via its other end by passing through the envelope via an opening, and also a tubular ring positioned on the inner surface of the envelope in the area of the opening and configured to rigidly connect the envelope to the body of the drive tool. The connector piece is configured to be able to pass through the ring and turn freely inside the ring when driven by the rotary drive tool. The connector piece, the ring and the envelope have means which interact in order to form a leaktight barrier between the inside and the outside of the envelope in the area of the opening.

United States Patent Publication No. 8944069 B2 discloses devices and methods which allow multiple use of non-sterile medical devices with sterile medical devices for performing medical procedures requiring sterile conditions.

### Summary

Although power tool covers that can be sterilized multiple times and are reusable may be useful, it may be beneficial to provide a cover system that is optimized for single use, as an alternative.

In some cases sterilization procedures, speed of sterilization equipment, or lack of convenient access to sterilization equipment, for example, may slow down access to care, such as access to a surgical procedure or other procedure requiring sterility. Having quicker access to sterile tools for procedures that require sterility could be advantageous. Quicker access to sterile surgical tools, for example, could be particularly advantageous during mass-causality or high-causality events, such as a natural disaster or a mass vehicle accident.

There is a general need for more cost effective ways to provide improved access to tools for use in surgery and other environments that require sterilization.

The invention is defined by the independent claims. The present disclosure relates to a number of aspects or embodiments. These include, without limitation:
- embodiments for providing a sterile barrier between a sterile field and a non-sterile power tool;
- embodiments for sealing a power tool within a cover while transmitting motion from the power tool to a drill bit, blade or other implement outside of the cover;
- embodiments for sealing covers;
- embodiments for packaging covers;
- embodiments for inserting non-sterile power tools inside sterile covers.

Such embodiments as disclosed herein include covers, cover systems, components, and methods.

One aspect of the present disclosure relates to a disposable cover system for a power tool. The cover system includes a cover that has a sterile outside surface and defines an inner cavity, an opening through which the power tool is insertable into the inner cavity, and an aperture through which a pass-through to transmit movement generated by the power tool from inside the inner cavity to outside the cover is extendable. The cover system also includes a closing mechanism to close the opening, and a compressible gasket surrounding the aperture.

A transmission component to transmit movement from a power tool through a sterile barrier is disclosed. The transmission component includes a first part to be positioned at a first side of the sterile barrier, a second part to be positioned at a second side of the sterile barrier opposite the first side, and a movable component. The second part is couplable with the first part to trap a portion of the sterile barrier between the first part and the second part and form a seal around an aperture in the sterile barrier. The movable component is coupled to the first part, the second part, or both the first part and the second part, to extend through the aperture and to be coupled to the power tool to transmit movement generated by the power tool through the sterile barrier.

An adapter to adapt a sterile barrier to a transmission component that is configured to transmit movement from a power tool through the sterile barrier. The adapter includes a first part to be positioned at a first side of the sterile barrier, and a second part to be positioned at a second side of the sterile barrier opposite the first side. The second part is couplable with the first part to trap and compress a portion of the sterile barrier between the first part and the second part and form a compression seal around an aperture in the sterile barrier.

A multi-part device includes a first part to be positioned at a first side of a sterile barrier, and a second part to be positioned at a second side of the sterile barrier opposite the first side. The second part is couplable with the first part to create an aperture in the sterile barrier and to trap a portion of the sterile barrier between the first part and the second part and form a seal around the aperture.

Yet another aspect of the present disclosure relates to a method that involves: orienting an opening of a disposable cover to receive a power tool into an inner cavity defined by the disposable cover, the disposable cover having a sterile outside surface and further defining an aperture; operating a closing mechanism to close the opening with the power tool inside the inner cavity with a drive part of the power tool adjacent the aperture; and forming a compression seal around the aperture to seal the power tool inside the inner cavity.

Further aspects and example embodiments are illustrated in the accompanying drawings and/or described in the following description.

### Brief Description of the Drawings

The accompanying drawings illustrate non-limiting example embodiments of the invention.
Figure 1A is a side view of a power tool system according to an example embodiment.
Figure 1B is an exploded view of the power tool system of Figure 1A.
Figure 1C is a schematic view of example individual components of the power tool system of Figure 1A.
Figure 2A is a schematic view of a disposable cover system according to an example embodiment.
Figures 2B and 2C are views from the left-hand side and right-hand side, respectively, of the disposable cover system of Figure 2A.
Figures 2D to 2F are views of disposable covers according to further example embodiments.
Figures 3A to 3E schematically illustrate example embodiments in which a portion of a cover is trapped between parts of a power tool system to form a seal around an aperture in the cover.
Figure 4A schematically illustrates a power tool system in which a portion of a cover trapped between a power tool and an example pass-through coupled to the power tool.
Figure 4B is a partial cutaway view of a power tool system similar to the power tool system of Figure 4A, in which a portion of a cover is trapped between a power tool and another example pass-through coupled to the power tool.
Figure 4C is a perspective view of the example pass-through shown in Figure 4A, which may be coupled to a power tool inside an inner cavity of a cover.
Figure 4D is a perspective view of an example adapter that may be coupled to a power tool.
Figure 5A is a perspective view of the example pass-through shown in Figure 4B, which may be directly coupled to a power tool inside an inner cavity of a cover.
Figure 5B is an exploded view of the example pass-through of Figure 5A.
Figure 5C schematically illustrates an embodiment in which a portion of a cover is trapped between example first and second portions of a pass-through that are coupled together.
Figures 6A and 6B are a perspective view and a side view, respectively, of an embodiment in which a portion of a cover is trapped between parts of an example adapter.
Figure 6C is a perspective view of an example nose mate of the adapter shown in Figures 6A and 6B.
Figures 6D and 6E are a perspective view and a top view, respectively, of an example nose of the adapter shown in Figures 6A and 6B.
Figure 6F is an exploded view of the adapter shown in Figures 6A and 6B.
Figure 7A is a top view of part of a multi-part device according to another embodiment.
Figures 7B and 7C are a top view and a side plan view, respectively, of another part of a multi-part device.
Figure 8A is a schematic view of a portion of a disposable cover system according to another embodiment.
Figure 8B is a schematic view showing an example interaction between a user's hands and a portion of the disposable cover system of Figure 8A.
Figures 9A to 9D are views illustrating use of a cover system according to an embodiment.
Figures 10A and 10B schematically illustrate attachments of straps to a cover according to example embodiments.
Figures 11A to 11F illustrate example folding sequences for folding a cover.
Figure 12 is a schematic perspective view of another example power tool system.
Figure 13 is an enlarged perspective view of an example assembly shown in Figure 12.
Figure 14A is a perspective view of an example adapter.
Figure 14B is a bottom view of the adapter of Figure 14A.
Figure 14C is a perspective view of the adapter of Figure 14A from an end of the adapter which is receivable in a receiving end of a power tool.

### Detailed Description

Throughout the following description, specific details are set forth and provided by way of example, in order to provide a more thorough understanding of illustrative embodiments the invention. However, embodiments of the invention may be practiced without these particulars. In other instances, well known elements have not been shown or described in detail to avoid unnecessarily obscuring embodiments of the invention. Accordingly, the specification and drawings are to be regarded in an illustrative, rather than a restrictive sense.

Surgical procedures typically require operating on a patient within a sterile environment or "field". Doing otherwise increases the likelihood of a patient experiencing post-surgical adverse effects such as surgical site infection or osteomyelitis.

In some cases a team of medical professionals, including surgeons, operating room nurses, and anesthesiologists, for example, travels to a community to perform a series of needed surgical procedures over a day, a week, several weeks, or another period of time. As described in, for example, international PCT application No. PCT/CA2015/050290 from which the above-referenced United States Patent No. 10,405,937 originated, a non-sterile power tool may be used for surgical procedures by inserting the non-sterile tool into a sterile cover that may withstand multiple sterilization cycles. Such a cover may be sterilized between different surgical procedures. Alternatively, the team may transport a separate sterilized cover for each procedure. With covers that are reusable, the used covers would typically be returned by the team for sterilization and reuse.

One aspect of the present disclosure relates to a disposable sterile cover that may be used to enclose a non-sterile power tool. The cover may be manufactured relatively inexpensively. The cover may also be packaged compactly into a disposable package. The package maintains sterility of the cover. A non-sterile tool may be quickly inserted into the cover. Following a surgical procedure the tool may be removed from the cover and the cover may be disposed of.

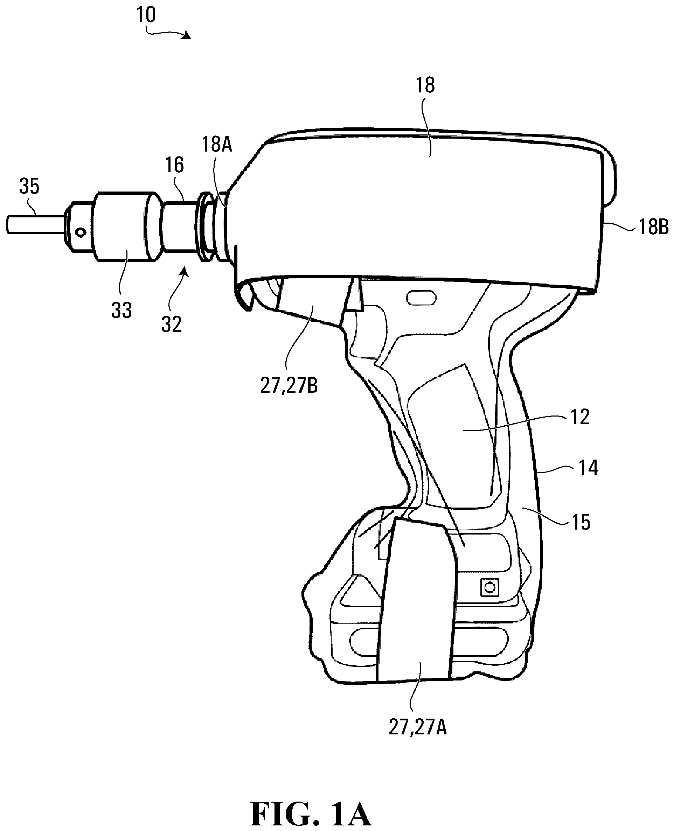

Figure 1A shows an example power tool system 10 according to an embodiment. The power tool system 10 may be used in surgery.

The power tool system 10 comprises a non-sterile power tool 12, such as a commercially available power drill, power screwdriver, driver, reciprocating saw, or oscillating saw. The power tool 12 may be selected to be a model that has characteristics related to a target or requirement for one or more parameters such as performance or safety, that make it appropriate to use for surgical procedures or other medical procedures.

The example power tool system 10 also includes a disposable sterile cover 14. The power tool 12 may be located inside an inner cavity 15 of the disposable sterile cover 14. The cover 14 provides a barrier between a sterile field and the power tool 12.

A pass-through 16 may be coupled to the power tool 12 inside the cover 14, and also extends from the inner cavity 15 that is defined by the cover 14 to outside the cover. The pass-through 16 transmits power or movement generated by the power tool 12 from the inner cavity 15 of the cover 14 to outside the cover 14. The power or movement may, for example, operate a drill bit, saw, another cutting implement, or another device such as a traction pin.

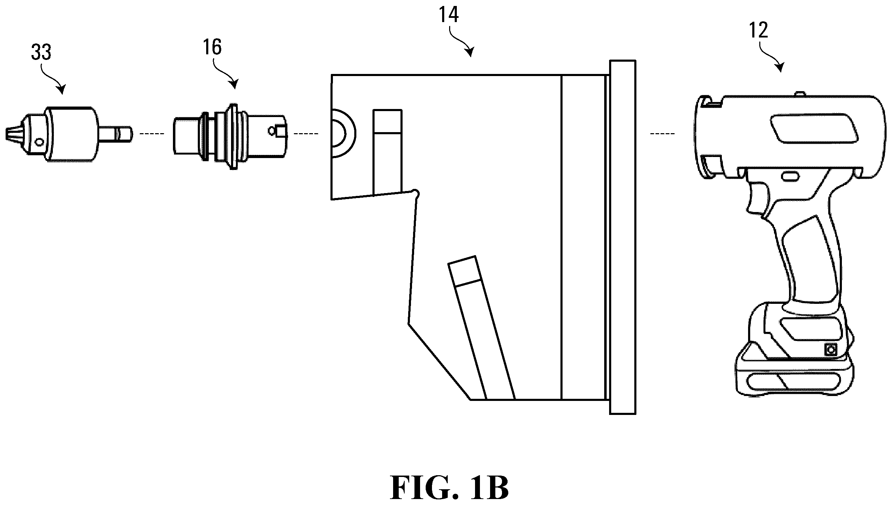

Figure 1B is an exploded view of the example power tool system 10. Figure 1B schematically shows individual example components which may be put together, in the manner generally illustrated by the exploded view in Figure 1B, to form the example power tool system 10 of Figure 1A. For completeness, it is noted that the secondary cover 18 shown in Figure 1A is not shown in Figure 1B. As disclosed elsewhere herein, installation of the secondary cover 18 may involve movement of the cover in multiple directions to cover multiple components that are shown in Figure 1B, and therefore the secondary cover 18 has not been shown in the exploded view in Figure 1B.

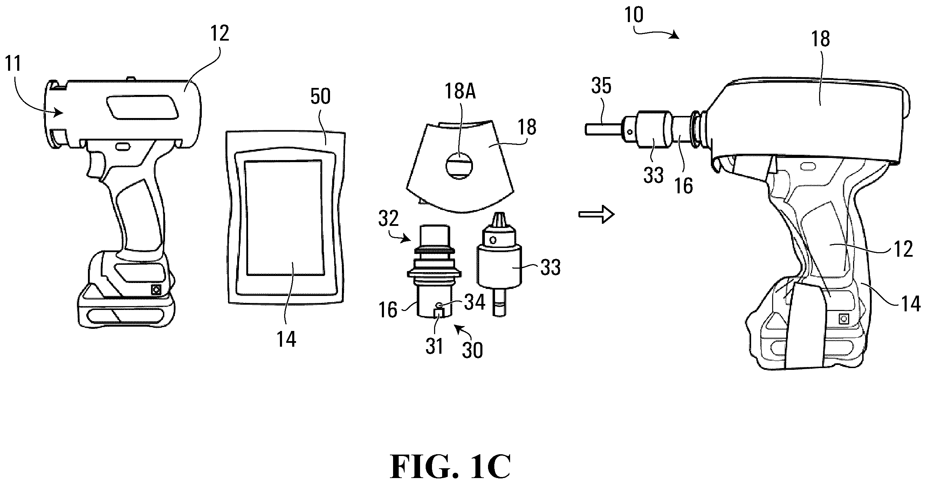

The exploded view in Figure 1B illustrates the various components of the example power tool system 10 in a state in which the components are ready to be assembled, whereas Figure 1C represents such components in a packaged or storage state. For example, with reference to the individual components as shown Figure 1C in the packaged or storage state, prior to or during assembly of a power tool system the cover 14 would be removed from packaging 50, and the cover and other components would be oriented relative to each other and coupled together as illustrated in the exploded view in Figure 1B to assemble the example power tool system 10 for use.

The power tool 12 may be a standard, off-the shelf power tool of the type that may be used by tradespersons or homeowners. Such tools are commonly available and sold under brand names such as, for example, Dewalt^{™}, Milwaukee^{™}, Bosch^{™}, Makita^{™}, Rigid^{™}, Black+Decker^{™} and Panasonic^{™}. Such standard power tools are very inexpensive in comparison to purpose-made surgical tools, but still can be reliable. Apart from the fact that they cannot be effectively sterilized using normal sterilization procedures, many standard power tools could be adapted, using a cover system as disclosed herein, for use in medical environments such as in an operating room having electromagnetic interference requirements for example, or the like. The power tool 12 may, for example, be a battery-powered rotary tool such as a drill or driver.

Where the power tool 12 is a rotary tool, one or more of the following characteristics may be desirable:
- operating speeds that match conventional surgical drills such as 300-1600 rpm for applications such as reaming and drilling;
- operating torques that match conventional surgical drills, such as 6-20 Nm;
- light weight, such as less than 1 kg;
- compact design, within one or more target physical dimensions;
- battery operated;
- robust, in terms of not being prone to requiring complex maintenance on a regular basis, being operable reliably for long periods without special maintenance, being durable for travel, or being able withstand rough handling, for example;
- usable in medical environments in which it is required to maintain compliance with one or more prescribed standards, such as not producing excessive electromagnetic disturbance, not operating at temperatures exceeding a prescribed threshold operating temperature, not exposing a patient to an excessive risk of electrical shock, being made of one or more particular materials, or not including one or more particular materials, for example.

The cover 14 is designed to enclose the power tool 12 during surgery. The cover 14 may be used to prevent non-sterile matter which may be inside the inner cavity of the cover from entering the sterile field. The cover 14 may also or instead be used to prevent matter, such as potentially damaging or hazardous liquids such as bio-fluids that may be outside the cover 14, from entering into the inner cavity 15.

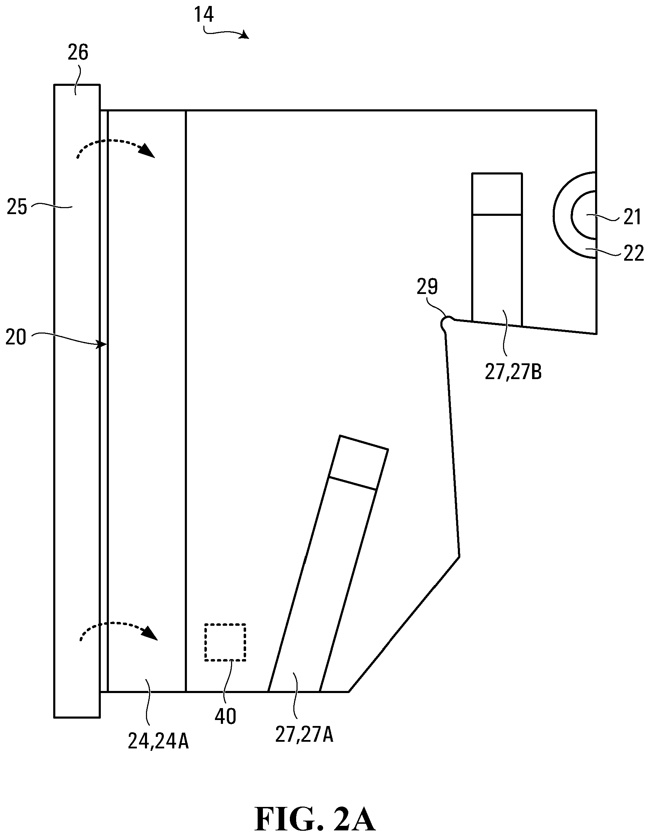

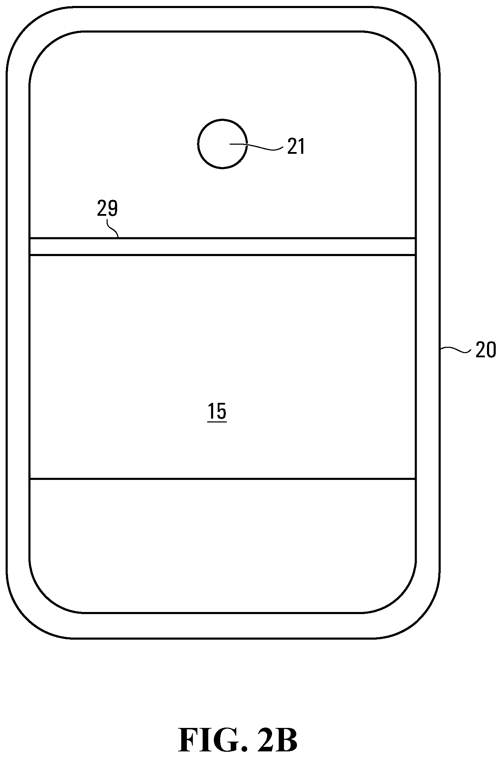

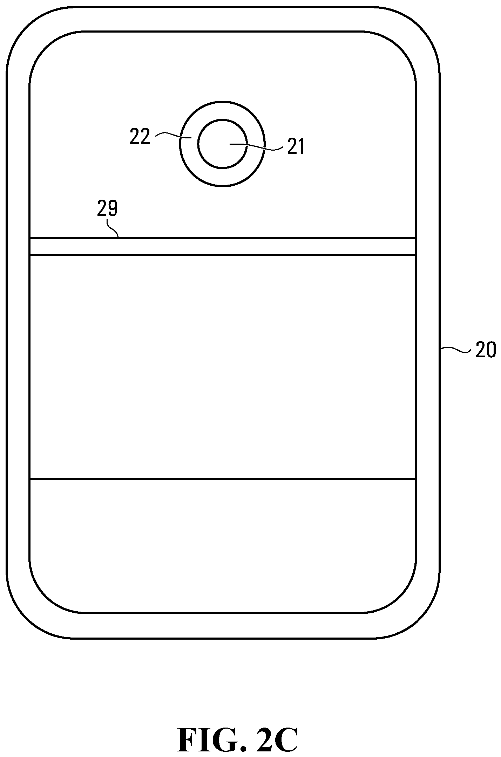

The cover 14 also, at least initially, provides access to the inner cavity 15 in order to allow insertion of the power tool 12 into the inner cavity and coupling of the pass-through 16 to the power tool 12. Figure 2A for example, which is a schematic view of a disposable cover system according to an example embodiment, shows that the cover 14 may comprise an opening 20 through which the power tool 12 may be inserted into inner cavity 15. This is perhaps more apparent from the view shown in Figure 2B. The pass-through 16 may extend through an aperture 21 of the cover 14, as may be more readily apparent from the view shown in Figure 2C. In Figure 2A, the cover 14 is shown in profile, as the cover would appear if folded flat. The views shown in Figures 2B and 2C are more closely representative of how the cover 14 would appear when opened for insertion of a power tool into the inner cavity.

Various other features that may be provided in some embodiments are also illustrated in Figures 1A to 2C and are described in detail elsewhere herein.

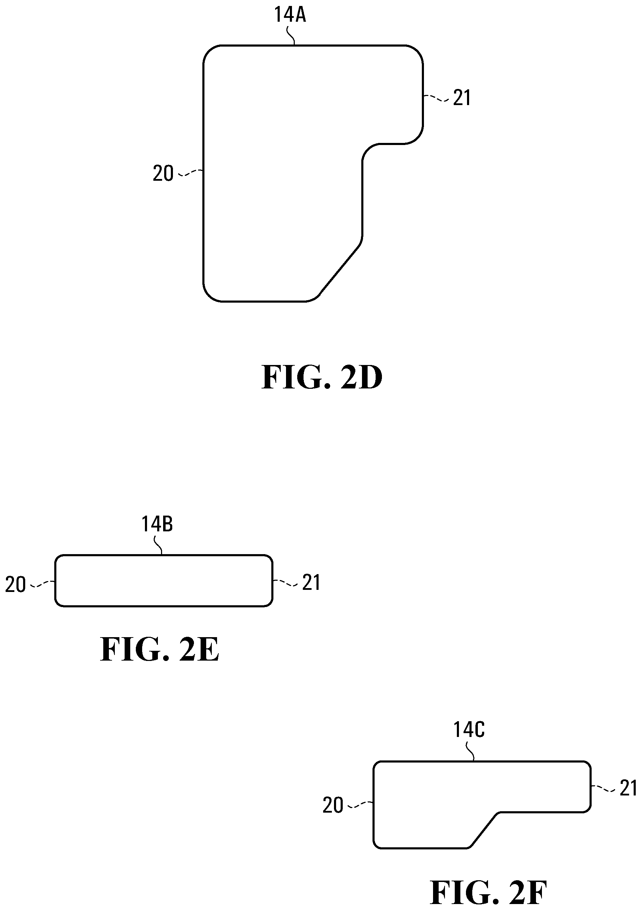

It should be appreciated that these drawings illustrate example embodiments. Other embodiments may, but need not necessarily, include all of the features shown. For example, Figures 2D to 2F are views of disposable covers according to further example embodiments. The views in Figures 2D to 2F are side profile views to illustrate different shapes and features that may be used or provided in other embodiments. Figure 2D illustrates a cover 14A that is similar to the cover 14 shown in Figure 2A, but with rounded corners. The example cover 14B shown in Figure 2E has a substantially rectangular or elongate shape, and may be suitable for a power screwdriver for example. In Figure 2F, the general shape of the example cover 14C is similar to that of the example cover system 14 in Figure 2D, but may be more suitable for a differently shaped power tool, such as a reciprocating saw.

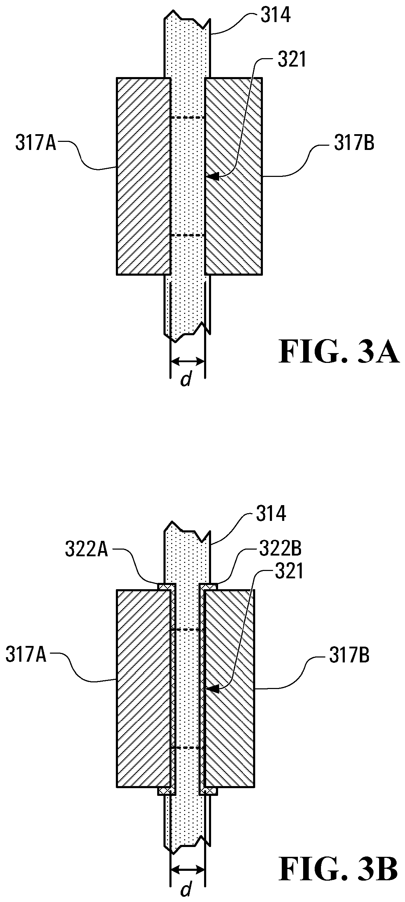

Considering first some example features related to an aperture as shown by way of example at 21 in Figure 2A, in some embodiments, a seal around such an aperture may be formed by trapping a portion of the cover which surrounds the aperture between opposing parts of a power tool system, opposing surfaces, or opposing compression members, as shown schematically in Figure 3A. For example, a portion of a cover 314 may be trapped between a first compression member 317A, inside an inner cavity defined by the cover, and a second compression member 317B that is outside the cover 314. The first compression member 317A and the second compression member 317B, and other compression members disclosed herein, are examples of compressible members that may be positioned or provided inside and outside a cover.

A cover is shown in Figure 3A and some other drawings with a different reference number than in Figures 1A to 2C, to illustrate that embodiments are not necessarily dependent upon any particular features of a cover. Different embodiments of a cover may include the same, similar, or different features. For example, the cover 14 shown in Figures 1A to 2F includes an aperture 21 and the cover 314 in Figure 3A also includes an aperture 321, but these covers 14, 214 may include different subsets of other features. More generally, features that are disclosed herein with reference to one embodiment may, but need not necessarily, be provided in other embodiments. This applies not only to covers, but also to other parts, components, systems, and methods disclosed herein.

The compression members 317A and 317B are configured to compress a portion of the cover 314 in a sealing region that extends completely around aperture 321 in some embodiments. Trapping the cover 314 between the compression members 317A and 317B as in the example shown compresses the cover 314 between the compression members, thereby forming the desired seal around the aperture 321.

In use, the cover 314 is placed between the compression members 317A and 317B with aperture 321 aligned inside the sealing region, and the compression members 317A and 317B are moved toward one another to effect a compression seal at least between second compression member 317B and the cover 314.

A power tool system such as the power tool 12 in the example power tool system 10 in Figure 1A is preferably designed to facilitate fast and easy changes of a cover, for example between different operations or during an operation if there is a sterile breach of the cover such as a puncture or tear. Any of various mechanisms may be used to effect a seal by advancing parts of one or more components, such as the compression members 317A and 317B in Figure 3A toward one another and to release a cover, after the cover has been used for example, by separating such parts as the compression members 317A and 317B.

In some designs, the compression member 317B is advanced towards the compression member 317A to effect a seal by a motion which results from coupling a pass-through to a power tool. Such motion may be perhaps most apparent from the exploded view in Figure 1B, in which the pass-through 16 would be moved toward and coupled to the power tool 12 during assembly of the example power tool system 10. In such designs, sealing of the cover 14 around an aperture 21, shown in Figures 2A and 2C for example, occurs automatically and simultaneously with the coupling of the pass-through 16 to the power tool 12.

With reference to Figure 3A, in some designs one or both of the compression members 317A and 317B are mounted to move relative to a pass-through such that sealing of the cover 314 around the aperture 321 may be done before, during or after coupling the pass-through to a power tool.

In some designs, one or both of the compression members 317A and 317B are designed to be coupled to one another to effect a seal to the cover 314 around the aperture 321 before a pass-through is coupled to a power tool. In such designs, a pass-through may be configured to be detachably coupled to one or both of the compression members 317B and 317A after the compression members 317A and 317B have been coupled to one another to seal around the aperture 321.

Engaging surfaces, such as surfaces of the compression members 317A, 317B that engage the cover 314, are preferably smooth. Additionally, engaging surfaces of the compression members 317A, 317B may be planar. Having smooth surfaces, planar surfaces, or surfaces that are both smooth and planar may advantageously increase reliability with which a continuous seal may be formed around the aperture 321 by compressing the cover 314 between the compression members 317A, 317B. In some embodiments an engaging surface of one or both of the compression members 317A and 317B comprises a protruding element such as a protruding ring, configured to indent, pierce, enclose, or otherwise deform, engage, abut, or cooperate with the cover 314 to improve the seal formed around the aperture 321. An element configured to pierce a cover may create or form an aperture in the cover at any location that is suitable or convenient, as described in further detail elsewhere herein.

In some embodiments one or more gaskets surround an aperture in a cover, as shown by way of example in Figures 2A and 2C as a gasket 22 surrounding the aperture 21. A gasket may be made of a compressible material. Trapping one or more gaskets such as the gasket 22, which may include compressing the gasket(s), may increase the likelihood of forming a continuous seal around an aperture. See Figures 3B to 3E for illustrative examples.

As shown by way of example in Figures 3B to 3E, a gasket may surround an aperture on either side of a cover. For example, with reference to Figure 3B, a gasket 322A may surround the aperture 321 inside the cover 314 and another gasket 322B may surround the aperture 321 outside the cover 314. In some embodiments a cover system comprises a single gasket either inside or outside a cover. Providing one or more gaskets on a single-use cover ensures that a fresh gasket will always be used to help seal around an aperture in the cover. In addition, or in the alternative, a gasket may be provided on one or both of the compression members 317A and 317B. More generally, a gasket may be attached to a cover or one or more other components of a cover system or power tool system, integrated with a cover or one or more other components of a cover system or power tool system, or provided separately from a cover and other components of a cover system or power tool system.

A gasket, or each gasket if more than one gasket is provided, may have any one or more of the following properties or features in some embodiments:
- be made from or include a closed-cell polyethylene foam;
- provide enough compression resistance to form a seal when compressed, between compression members such as the compression members 317A, 317B for example;
- be biocompatible;
- be hydrophobic;
- be manufactured inexpensively, for example by using a die to punch a plurality of gaskets from a sheet of material or by using a molding process.

In some embodiments a gasket has an inner diameter of less than 35 mm. In some embodiments a gasket has an inner diameter between 20 mm and 30 mm, such as 29.5 mm. **In** some embodiments a gasket has an outer diameter of less than 60 mm. In some embodiments a gasket has an outer diameter between 40 mm and 55 mm, such as 49.5 mm. In some embodiments a gasket is less than 10 mm thick. In some embodiments a gasket is between 5 mm and 10 mm thick, such as 6.4 mm thick before it is compressed.

A gasket may be circular, as shown by way of example in Figure 2C, but non-circular gaskets may be used in some embodiments. The shape of a gasket may match a shape of an aperture. Gasket size may also or instead be related to aperture size, with an inner diameter or dimension of a gasket matching a diameter or dimension of an aperture. in other embodiments, an inner diameter or dimension of a gasket is larger or smaller than a diameter or dimension of an aperture.

An aperture in a cover may be circular, as shown by way of example in Figure 2C, but this is not necessary. In some embodiments an aperture has a diameter of less than 35 mm. In some embodiments an aperture has a diameter between 15 mm and 30 mm, such as 29.5 mm. In some embodiments an aperture is punched out of a cover with a die. In other embodiments, an aperture is created or formed in the cover when the cover is to be used with a power tool.

In embodiments that include multiple gaskets, each gasket 22 may be the same or different.

A gasket may be bonded or otherwise attached to a cover. For example, a gasket may be bonded to a cover using double sided tape, a suitable adhesive, heat welding, or the like.

In embodiments that include one or more gaskets, a part, component, or mechanism that supports a compression member 317A or 317B may be designed so that when the compression members are fully advanced toward one another, the compression members are separated from one another by a distance d. Distance d is small enough that a portion of a cover that defines an aperture will be compressed between the compression members sufficiently to seal but will not be over compressed. In some embodiments, distance d is 10 mm or less. In some embodiments distance d is 5 mm or less. In some embodiments distance d is 1 mm or less.

The compression members 317A, 317B may comprise one or more surfaces of any of various parts or components of power tool system. With reference to the exploded view in Figure 1B for example, a compression member may be or include one or more surfaces of a body of the power tool 12 that is to be positioned inside the inner cavity of the cover 14, or the pass-through 16. A compression member may be or include one or more surfaces of another power tool system component disclosed herein, such as an adapter configured to receive a pass-through.

Figures 3B to 3E illustrate several embodiments that include gaskets.

In Figure 3B, gaskets 322A, 322B are provided on both sides of the cover 314, and are compressed by the compression members 317A, 317B to form a compression seal around the aperture 321. Both the cover 314 and the gaskets 322A, 322B are compressed in this example, within the distance d between the compression members 317A, 317B.

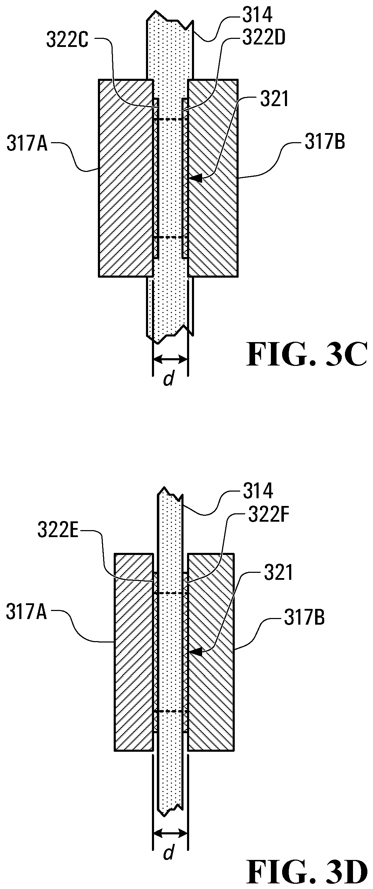

Figure 3C illustrates an embodiment in which gaskets 322C, 322D are provided on both sides of the cover 314, but the gaskets do not extend as far along surfaces of the cover as in the example shown in Figure 3B. The gaskets 322C, 322D are compressed by the compression members 317A, 317B to form a compression seal around the aperture 321, and as in Figure 3B both the cover 314 and the gaskets are compressed within the distance d between the compression members 317A, 317B. In Figure 3B, the gaskets 322A, 322B, at least when compressed, extend beyond the compression members 317A, 317B, whereas the gaskets in 322C, 322D in Figure 3C do not extend beyond the compression members.

A further example is shown in Figure 3D. The example in Figure 3D is similar to the example in Figure 3C, with the exception that only the gaskets 322E, 322F in Figure 3D are compressed by the compression members 317A, 317B to form a compression seal around the aperture 321. The cover 314 is not compressed in this example.

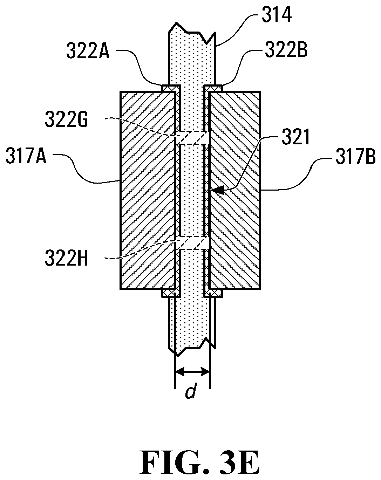

The example shown in Figure 3E is illustrative of an embodiment in which a gasket, or a separate gasket, extends axially in the aperture 321. In Figure 3E, such axial extension is illustrated at 322G, 322H. In the case of circular aperture, axial extension of a gasket may be in the form of a tubular or cylindrical structure, for example. An axial gasket may be used in conjunction with radially extending gaskets such as 322A, 322B, or a gasket may include an axially extending portion at 322G, 322H and one or more radially extending portions at 322A, 322B. In other words, axially extending portions 322G, 322H may be or include one or more separate gaskets, or different parts of a single gasket.

In the example shown in Figure 3E, the gasket(s) 322A, 322B, 322G, 322H and the cover 314 are compressed within the distance *d* by the compression members 317A, 317B. In other embodiments, the compression members 317A, 317B compress only the gasket(s) 322A, 322B, 322G, 322H and not the cover 314.

A compressible gasket that is substantially flat when uncompressed may extend axially into an aperture in a cover when compressed. With reference again to Figure 3E, the portions 322G, 322H may be formed when a gasket is compressed, or may include parts of multiple compressed gaskets, in embodiments in which the aperture 314 is smaller than an opening or aperture in the gasket(s). **In** such embodiments, compression of the gasket(s) may deform the gasket(s) to such a degree that the gasket(s) will extend at least partially into the aperture 314, potentially providing an improved seal.

Various embodiments of a cover system are described above with reference to Figures 1A to 3E. These embodiments are illustrative of a disposable cover system for a power tool. Such a disposable cover system may include a cover or enclosure such as 14, 314 that may include a sterile outside or outside surface, and define an inner cavity inside the cover, shown by way of example at 15, dimensioned to receive the power tool. The cover also defines an opening such as 20 through which the power tool is insertable into the inner cavity. The opening is dimensioned to allow insertion of the power tool into the inner cavity. The cover also defines an aperture or second opening such as 21, 321 through which a pass-through to transmit movement generated by the power tool from inside the inner cavity to outside the cover is extendable. The aperture or second opening is dimensioned to allow the pass-through to be inserted into the inner cavity and to be coupled to the power tool. In some embodiments the pass-through comprises an outside end outside of the cover or enclosure and has a coupling mechanism operable to coupled a tool or instrument to the outside end. The pass-through 16 is an example of a pass-through, and other examples are provided herein.

A cover system may also include a closing mechanism or closure arranged to close the opening, and a compressible gasket surrounding the aperture. A compressible gasket is shown generally as a gasket 22, and additional examples are shown in Figures 3A to 3E.

A compressible gasket may be attached to a cover, or the cover itself may include the compressible gasket. A compressible gasket may be integrated with the cover, by providing a gasket between layers of a cover for example.

In some embodiments, a cover system includes a sterile compressible gasket disposed on the sterile outside surface of the cover, as shown by way of example at 22 in Figure 2A. A further compressible gasket may be disposed on an inside surface of a cover inside the inner cavity, surrounding the aperture. The examples shown in Figures 3B to 3E all include a compressible gasket disposed on opposite surfaces of the cover 314. One of these surfaces would be an inside surface, and the other would be a sterile outside surface. As described elsewhere herein, compressing such gasket(s), between first and second compression members for example, forms a seal around the aperture.

As shown by way of example in Figure 3E, a compressible gasket may extend axially in the aperture. A compressible gasket may further extend radially from the aperture along the sterile outside surface of the cover, and may also or instead further extend radially from the aperture along an inside surface of the cover inside the inner cavity. In an embodiment consistent with Figure 3E, a single gasket may extend axially in the aperture 321 and radially along opposite inside and outside surfaces of the cover 314.

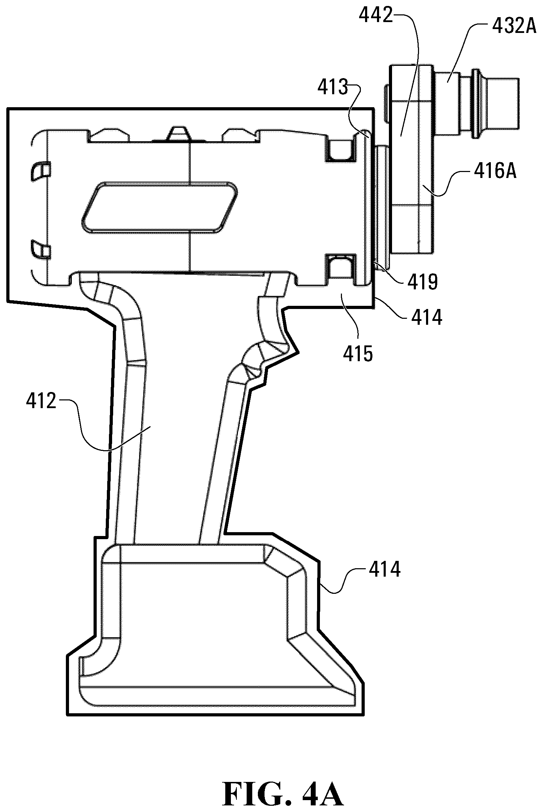

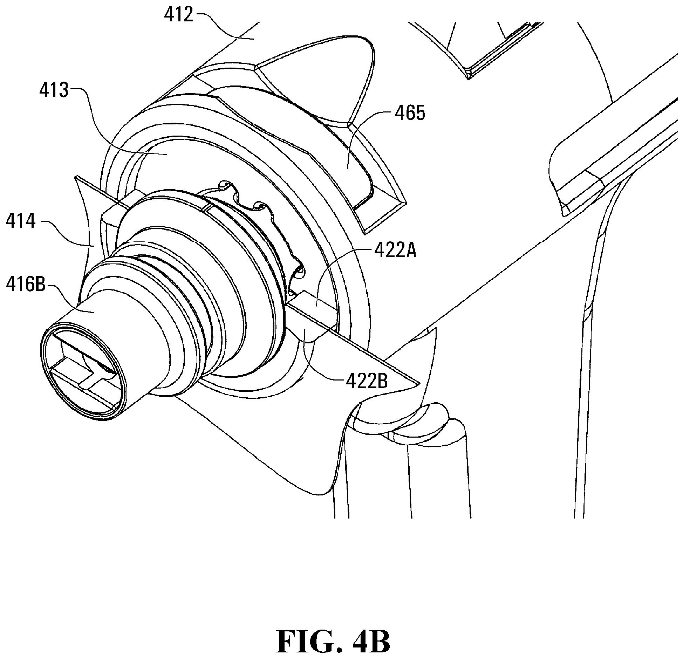

In some embodiments, a pass-through is directly coupled to a power tool. In such embodiments, as shown by way of example in Figures 4A and 4B, a portion of a cover 414, and gaskets 422A, 422B if included as shown in the example in Figure 4B, may be trapped between a surface 413 of a power tool 412 inside an inner cavity 415 defined by the cover and a surface 419 of a pass-through 416A or 416B that is outside of the cover 414. In such embodiments, a first compression member may be provided by the surface 413 and a second compression member may be provided by the surface 419. This is illustrative of one possible embodiment of the first and second compression members 317A, 317B shown in Figures 3B to 3E.

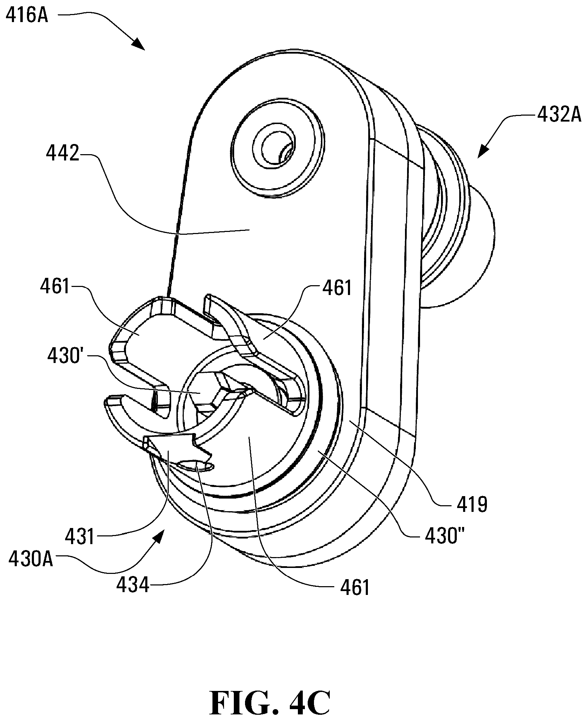

In some embodiments, a pass-through is as shown by way of example as pass-through 416A in Figures 4A and 4C, which may be directly coupled to a coupling mechanism of the power tool 412. The illustrated offset of outside portion 432 relative to an axis of motion of the power tool 412 is optional. Another example of a pass-through, without an offset, is shown at 416B in Figure 4B. Both of these pass-throughs 416A, 416B represent illustrative and non-limiting embodiments of a pass-through 16 shown more generally in Figures 1A-1C.

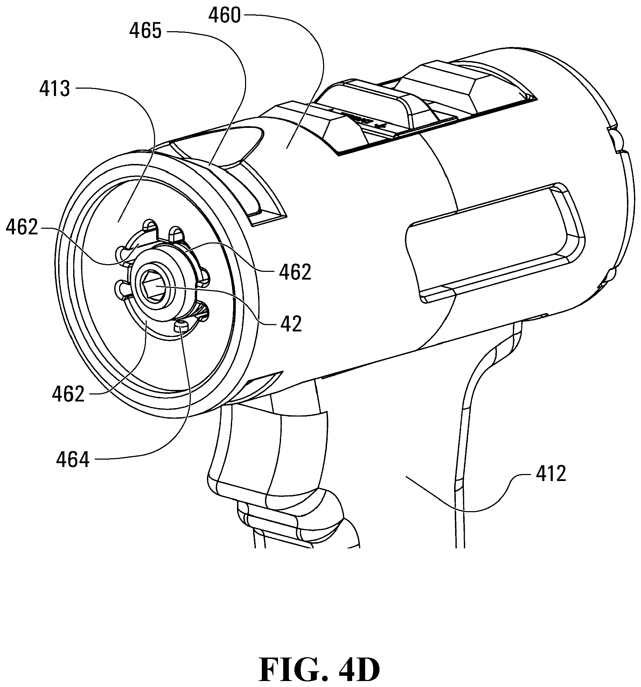

The example "offset" pass-through 416A comprises an inner portion 430A which may be coupled to a coupling mechanism of the power tool 412. The inner portion 430A may be inserted into the inner cavity 415 through an aperture in the cover 414 and coupled to a coupling mechanism of the power tool 412. The inner portion 430A may comprise an anchoring portion configured to secure and/or orient the pass-through 416A to the power tool 412 and a rotating portion configured to transmit, from the inner cavity to outside the cover through the aperture, movement that is generated by the power tool 412. In some embodiments a shaft 430' of the pass-through 416A may be coupled directly to a coupling mechanism of the power tool 412, such as a coupling mechanism 42 shown in Figure 4D. Once coupled to the power tool 412, the inner portion 430A may transmit power or movement generated by the power tool 412 inside the inner cavity 415 to an outer portion 432A of the pass-through 416A.

Additionally, coupling the pass-through 416A to the power tool 412 may automatically position the surface 419 of the pass-through a sufficient distance, such as a distance d shown in Figures 3A to 3E, away from the surface 413 of the power tool 412 to form a seal around an aperture in the cover 414. The inner portion 430A may comprise a gasket or O-ring 430" that is engageable with one or more gaskets 422A, 422B, and also or instead with the cover 414. A gasket 422A, 422B or O-ring 430" may help to ensure that a complete seal will be formed around an aperture in the cover 414.

The inner portion 430A may be configured to form a "push and click" coupling mechanism with the power tool 412. The inner portion 430A may, for example, be pushed into a receiving end of the coupling mechanism of the power tool 412. Either or both of the inner portion 430A and the receiving end of the power tool 412 may comprise features, such as facets, splines, ridges, or grooves, configured to orient the inner portion 430A relative to the receiving end. Additionally, or alternatively, either or both of the inner portion 430A and/or the receiving end of the power tool 412 may comprise one or more features configured to lock the pass-through 416A relative to the receiving end once the inner portion 430A has been inserted into the receiving end by a desired amount.

Inserting the pass-through 416A into the receiving end of the power tool 412 may, for example, slide a locking pin, such as a pin 464 shown in Figure 4D, of the receiving end along a ramp 431 shown in Figure 4C. Once the inner portion 430A has been sufficiently inserted, the locking pin may fall or be forced into a recess 434 of the inner portion 430A thereby locking the pass-through 416A relative to the receiving end of the power tool 412. In some embodiments the ramp 431 is configured to require the inner portion 430A to be pushed into the receiving end and twisted prior to the inner portion 430A being locked relative to the receiving end of the power tool 412. Such twisting may be clockwise or counterclockwise, or include both clockwise and counterclockwise twisting.

In some embodiments the inner portion 430A may be inserted into a standard coupling mechanism of the power tool 412, such as a chuck or a shaped recess of a power screw-driver as shown by way of example as the coupling mechanism 42 shown in Figure 4D.

In some embodiments the coupling mechanism of the power tool 412 may be modified to allow for direct insertion of the pass-through 416A.

In some embodiments the coupling mechanism of the power tool 412 is modified to match the coupling mechanism of a purpose-made surgical power tool to allow for direct coupling of surgical-grade tools to the power tool 412. In some embodiments the coupling mechanism of the power tool 412 or the pass-through 416A allows for direct coupling of commercially available tools available from companies such as Hudson^{™}, Zimmer^{™}, Stryker^{™}, Hall-Jacobs^{™}, AO^{™} or Synthes^{™}.

In some embodiments the coupling mechanism of the power tool 412 comprises a "male" type shaft and the inner portion 430A comprises a "female" type receiving end. In some embodiments the coupling mechanism of the power tool 412 may comprise an n-point coupling configured to receive a corresponding n-point shaft of the pass-through 416A. In some embodiments the shaft has a hexagonal cross-section. In some embodiments the shaft has a dodecagonal cross-section. In some embodiments the coupling mechanism of the power tool 412 comprises an n-point shaft and the pass-through 416 comprises an n-point recess configured to receive the n-point shaft.

In some embodiments the coupling mechanism of the power tool 412 is modified by coupling an adapter to the power tool 412. The adapter may comprise one or more features configured to position and/or secure the pass-through 416A relative to the coupling mechanism of the power tool 412.

Figure 4D shows an example adapter 460 coupled to the power tool 412. In the illustrated embodiment, the power tool 412 is a commercially available driver. Example adapter 460 may be positioned over an upper portion of the power tool 412. The adapter 460 is preferably securely coupled, for example non-movably coupled, to the power tool 412. For example, the adapter 460 may be bonded to the power tool 412. As another example, the adapter 460 may be made of at least a semi-stretchable material. In such embodiments opposing ends of the adapter 460 may be stretched over a portion of the power tool 412 thereby securely attaching the adapter 460 to the power tool 412. As another example, the adapter 460 may be rigid. In some embodiments the adapter 460 may be fastened to the power tool 412.

In some embodiments the adapter 460 comprises two portions which may be coupled together. Coupling the two portions together, by bonding the two portions together or by fastening the two portions together for example, may secure the adapter 460 to the power tool 412.

The adapter 460 may comprise one or more recesses 462 configured to receive guiding protrusions 461 of the pass-through 416A. In some embodiments the pass-through 416A comprises a single guiding protrusion 461. In some embodiments the pass-through 416A comprises a plurality of guiding protrusions 461. Insertion of the protrusions 461 into corresponding recesses 462 of the adapter 460 may position the pass-through 416A into a desired orientation relative to the power tool 412. The recesses 462 may comprise filleted corners. The fillets may, for example, facilitate easier insertion of the protrusions 461 into the recesses 462. In some embodiments, the recesses 462 are dimensioned to frictionally engage surfaces of the protrusions 461. Frictionally engaging surfaces of protrusions 461 may increase a strength of the coupling of the pass-through 416A to the power tool 412. In some embodiments the recesses 462 are formed between surfaces of the adapter 460 and opposing surfaces of an existing coupling mechanism of the power tool 412, such as the coupling mechanism 42.

Although the example adapter 460 comprises recesses 462, this is not mandatory. In some embodiments the adapter 460 comprises guiding protrusions which may be inserted into recesses of the pass-through 416A. In some embodiments the adapter 460 comprises at least one recess and at least one protrusion.

Engagement of the pin 464 with, for example, the ramp 31 of the pass-through 416A as the pass-through is coupled to the power tool 412 depresses the pin 464 until a hole 434 is proximate to the pin 464. Upon the hole 434 being positioned above the pin 464, the pin 464 may extend into the hole 434 thereby securing the pass-through relative to the power tool 412. In some embodiments the pin 464 is spring-loaded or otherwise biased in an inward radial direction.

The pass-through 416 may be uncoupled from the power tool 412 by uncoupling the pin 464 from the hole 434 and pulling the pass-through 416A outwards relative to the power tool 412 and the adapter 460. The adapter 460 may, for example, comprise a release mechanism 465 configured to uncouple the pin 464 from the hole 434. In some embodiments depressing the release mechanism 465 retracts the pin 464 thereby allowing for the pass-through 416A to be uncoupled from the power tool 412. In some embodiments the release mechanism 465 is recessed relative to surrounding portions of the adapter 460 to prevent accidental decoupling of the pass-through.

The surface 413 of the adapter 460 may be recessed relative to an outer edge of the adapter 460. Providing a recessed surface 413 may assist with aligning the gasket 422A, a nose mate described elsewhere herein, or another part or component with the adapter 460, potentially increasing the reliability with which a complete seal can be formed around an aperture in the cover 414.

A tool such as a drill bit, a saw, a wire, a reamer, or a traction pin, for example, may be coupled to the outer portion 432A of the pass-through 416A. An example of a tool is shown at 35 in Figure 1A. Preferably, different tools may easily be coupled and uncoupled to the outer portion during a surgical procedure.

The outer portion 432A of the pass-through 416A may comprise a chuck or a similar mechanism to releasably couple the pass-through a tool that is to be driven by the power tool 412. With reference to Figure 1A, for example, a chuck is shown at 33. Tightening the chuck 33 may couple a tool 35 to the outer portion 32 of the pass-through 16. Loosening the chuck 33 may uncouple the tool 35 from the outer portion 32 of the pass-through 16. As another example, the outer portion 32 of the pass-through 16 may comprise a recess having a particular cross section or guiding features. A male mating end of the tool 35 may have a matching cross-section. Inserting the male mating end into the recess may couple the tool 35 with the outer portion 32 of the pass-through 16. In some embodiments the tool 35 comprises a female mating end which may be pushed over a male receiving shaft of the outer portion 32.

Different tools 35 coupled to the outer portion 32 of the pass-through 16 may require different types of movement to be actuated. For example, a drill bit may require the outer portion 32 to rotate. In contrast, a reciprocating saw blade may require reciprocating linear or orbital movement of the outer portion 32. Different embodiments of the pass-through 16 may be used based on the type of tool 35 that is to be coupled to the outer portion 32 of the pass-through 16 and the type of power tool that is provided inside the cover 14. Some embodiments of the pass-through 16 transmit rotational movement to the outer portion 32.

Some embodiments of the pass-through 16 convert rotational movement generated by the power tool 12 into linear movement. Any of a wide variety of mechanisms may be provided to create reciprocating linear movement. For example, the pass-through 16 may include a slider crank mechanism, a cam and follower mechanism, a wobble plate mechanism, a reversing screw mechanism, or any other known mechanism for converting rotary motion to reciprocating linear motion.

Some embodiments of the pass-through 16 transmit linear movement from the inner portion to the outer portion 32. For example, the pass through 16 may comprise a shaft that is free to reciprocate. The shaft may slide in a linear bearing or bushing of the pass-through 16, and may also or instead be connected to a diaphragm that allows a desired or requisite degree of linear motion.

In some embodiments the pass-through 16 transmits oscillating linear movement, for a reciprocating saw for example. In some embodiments the pass-through 16 transmits oscillating circular movement, for an oscillating/sagittal saw or for a Tibial Plateau Leveling Osteotomy (TPLO) saw, for example.

In some embodiments the pass-through 16 is a universal power transmission component or movement transmission component. "Universal" means that a single pass-through 16 may be used during a surgical procedure to transmit two or more different types of movement to the outer portion of the pass-through 16. In some such embodiments the pass-through 16 may be switched between a first mode in which rotational movement of the inner portion is transmitted to the outer portion and a second mode in which rotational movement of the inner portion is converted into linear or side-to-side movement. Modes of such a pass-through 16 may be changed, for example, by turning a dial on the pass-through 16 or operating a switch, for example.

In some cases, the pass-through 16 transmits movement generated by the power tool 12 to a fitting in the outer portion 32 that is outside of the cover 14. Throughout a surgical procedure, different adapters, such as adapters that convert rotational movement into linear movement or adapters that convert rotational movement into oscillating circular movement, for example, may be coupled to outer portion 32 of the pass-through 16. Advantageously, this may facilitate different tools 35, such as drill bits, saw blades, or traction pins, to be used with the power tool 12 without breaking the sterile barrier. For example, enabling multiple adapters to be coupled to and decoupled from the outer portion 32 of the pass-through 16 may avoid breaking the seal around an aperture in the cover 14 to uncouple and couple different embodiments of the pass-through 16 to allow different types of tools to be driven by the power tool 12.

The pass-through 16 is optionally disposable. In such embodiments, a disposable pass-through may be provided with the cover 14. In some embodiments, the cover 14 may be delivered with a disposable pass-through pre-attached to the cover 14.

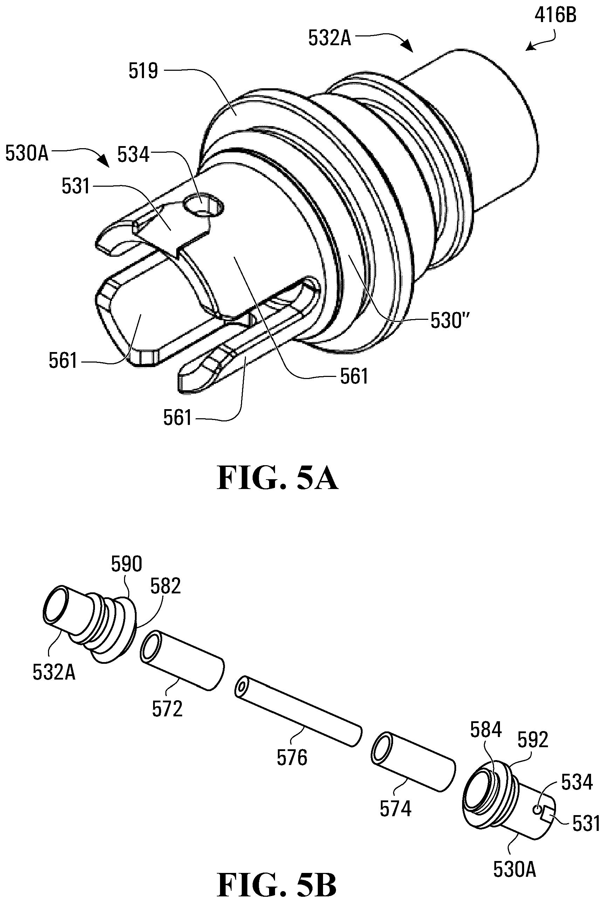

These and other features associated with a pass-through 16 may be implemented in conjunction with any of various types of pass-through. In some embodiments, for example, an axis of motion of an outside portion of a pass-through 16 can be offset relative to an axis of motion of a power tool 12. Referring again to Figure 4C, the inner and outer portions 430A and 432A of the example pass-through 416A are axially offset from one another. Offset mechanism 442 may be configured to axially offset the outer portion 432A from the inner portion 430A while transmitting movement from the inner portion 430A to the outer portion 432A. However, this is not necessary in all embodiments. In some embodiments the inner and outer portions of a pass-through are axially aligned with one another, as shown by way of example in Figure 5A, which is described in further detail below.

The offset mechanism 442 may provide a geared system, a belt system, or the like to transmit movement from the inner portion 430A to the outer portion 432A. The outer portion 432A may comprise a collet. Offsetting the outside portion 432A from an axis of motion of the power tool 412 may allow the collet to hold a longer wire (or K-wire) than could be held by the collet if the outside portion 432A were not offset from the axis of motion of the power tool 412. The offset mechanism 442 may remain stationary relative to one or both of the inner portion 430A and outer portion 432A.

The pass-through 416B shown schematically in Figure 5A is another embodiment of a pass-through. The pass-through 416B may be substantially identical to the pass-through 416A described elsewhere herein, except that inner and outer portions 530B and 532B of the pass-through 416B may be axially aligned and directly coupled together, or constructed to allow them to be directly coupled to and uncoupled from one another, rather than being offset from each other and coupled together through an offset mechanism in the case of the offset pass-through 416A. Elements 519, 530", 531, 534, and 561 in Figure 5A may be substantially the same as the similarly-labelled elements of the pass-through 416A. The pass-through 416B may also or instead include other pass-through features that are described herein, with reference to the pass-through 16 in Figure 1A, for example.

Figure 5B illustrates an exploded view of the pass-through 416B, with the inner portion 530A at the right-hand side of the drawing and the outer portion 532A at the left-hand side of the drawing. Figure 5B provides an example of an embodiment in which a pass-through has multiple part or components that may be releasably coupled together. Although the pass-through 416B includes multiple parts that may be coupled together and decoupled from each other in some embodiments, in other embodiments a pass-through of the form shown at 416B need not necessarily include multiple parts that are intended to be separable or decouplable.

In Figure 5B, 590 and 592 illustrate surfaces of the inner portion 532A and the outer portion 530A, respectively, that oppose each other to capture a portion of a cover when the pass-through 416B is assembled with the inner portion and the outer portion on opposite sides of the cover. A compression seal is formed in some embodiments, and accordingly the surfaces 590, 592 are further examples of compression surfaces or compression members.

Bearings 572, 574 are shown as an example of components that may isolate the inner portion 530A and the outer portion 532A from rotation of a shaft 576 by a power tool. The shaft 576 may be rotated or reciprocated, for example, to transmit movement from a power tool inside a cover to outside the cover, without also causing movement of the inner portion or the outer portion.

The inner portion 530A may include an anchoring portion, with a ramp 531 and a hole 534 in the example shown, to secure and/or orient the pass-through 416B to a power tool. The bearings 572, 574 and the shaft 572 are illustrative of a transmission portion that is configured to transmit power or movement from the inner cavity to outside the cover through an aperture. Although not specifically shown in Figure 5B, the shaft 576 may have a non-circular shape, such as a hexagonal cross-section, a dodecagonal cross-section, or an n-point cross-section.

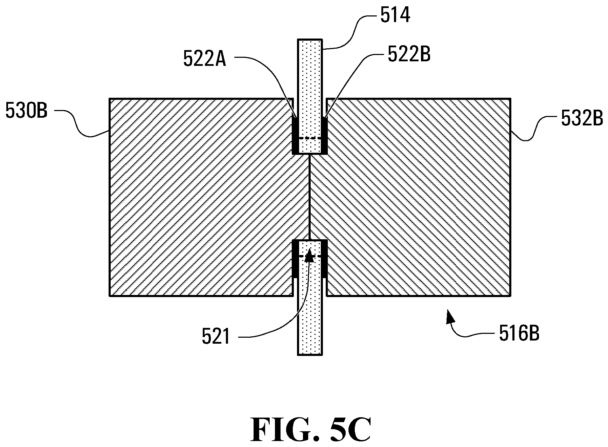

A multi-part pass-through such as the pass-through 416B may be useful, for example, to form a seal around an aperture in a cover using the pass-through itself. In such embodiments, as shown by way of example in Figure 5C, a portion of a cover 514 may be trapped between inner and outer portions 530A and 532A of the pass-through 416B. For example, coupling the inner portion 530A of the pass-through 416B to the outer portion 532A of the pass-through 16B may trap a portion of the cover 514 between the inner and outer portions 530A and 532A. Opposing surfaces of the inner and outer portions 530A and 532A, shown by way of example at 590, 592 in Figure 5B, may be a sufficient distance apart, for example a distance d as referenced above, to form a seal around an aperture 521 in the cover. The inner and outer portions 530A and 532A may be coupled to one another, for example, using one or more of a threaded coupling, a magnetic coupling, a bayonet coupling, a clip retention coupling, a spring-loaded ball coupling, a spring-loaded bar coupling, a spring-loaded pin coupling, and a spring retention coupling. With reference again to Figure 5B, components 582, 584 represent threaded columns, shafts, or tubes to couple the inner and outer portions 530A, 532A together via a threaded coupling. An outer surface of the column 584 is threaded in the example shown, to mate with a threaded inner surface of the column 582. Other types of threaded couplings, and other types of coupling including non-threaded couplings, are also possible. Other examples of couplings are provided elsewhere herein.

Although Figures 5A to 5C illustrate a multi-part linear pass-through 416B, an offset pass-through may also or instead be implemented using multiple parts that can be coupled with and decoupled from each other. With reference to Figure 4C, for example, the inner portion 430A could be releasably couplable with the offset mechanism 442, to capture a portion of a cover therebetween. In another embodiment, the offset mechanism 442 is also or instead releasably couplable with the outer portion 432A.

Multi-part pass-throughs may include other pass-through features disclosed herein.

A multi-part pass-through, whether linear or offset, is an example of a transmission component to transmit movement from a power tool through a sterile barrier. A multi-part transmission component may include: an inner portion such as 430A, 530A, or more generally a first part, to be positioned at a first side of the sterile barrier; an outer portion such as 432A, 532A, or more generally a second part, to be positioned at a second side of the sterile barrier opposite the first side; and a movable or drivable component such as the shaft 430', 576.

The second part is couplable with the first part to trap a portion of the sterile barrier between the first part and the second part and form a seal around an aperture in the sterile barrier. This is shown perhaps most clearly by way of example in Figure 5C.

The movable component is coupled to the first part, the second part, or both the first part and the second part, to extend through the aperture and to be coupled to the power tool to transmit movement generated by the power tool through the sterile barrier. With reference to Figures 5B and 5C, the shaft 576 is an example of such a movable part that would be coupled to the inner portion 530A and the outer portion 532A by the bearings 572, 574 at least when the pass-through 416B is assembled, and in an assembled state as shown by way of example in Figure 5C the shaft would extend through a sterile barrier that includes the cover 514 and gaskets 522A, 522B.

In some embodiments, a single bearing or other component may be used to couple a movable component to only part of a mufti-part transmission component. For example, one bearing 572, 574 could potentially be sufficient to support the shaft 576.

In an unassembled state, a movable component such as the shaft 576 may be coupled to either one of the parts of a multi-part transmission component. With reference to Figure 5B as an example, the bearings 572, 574 may be attached to or part of the outer portion 532A and the inner portion 530A, respectively, and the shaft 576 may be coupled to and held in one of the bearings prior to assembly of the pass-through 416B. The shaft would then be inserted into the other bearing during assembly.

The first part of a multi-part transmission component may include or provide a first compression surface, and the second part may include or provide a second compression surface, to engage and compress the portion of the sterile barrier at the first side and the second side, respectively. In such an embodiment, the seal is or includes a compression seal between the first compression surface and the second compression surface. The surfaces 590, 592 in Figure 5B represent examples of such compression surfaces.

In some embodiments, a transmission component includes one or both of: a compressible gasket on the first compression surface, and a compressible gasket on the second compression surface. One or more compressible gaskets may be provided separately, or, for example, the portion of the sterile barrier may include a compressible gasket for compression between the first compression surface and the second compression surface.

The first part and the second part may be couplable together via any of various types of couplings. For example, the first part and the second part may include respective cooperating components of one or more of: a threaded coupling, a press fit coupling, a magnetic coupling, a bayonet coupling, a clip retention coupling, a spring-loaded ball coupling, a spring-loaded bar coupling, a spring-loaded pin coupling, and a spring retention coupling to couple the second part with the first part.

The movement that is transmitted by a transmission component may be or include rotational movement generated by the power tool, in which case the movable component may be coupled to the first part, the second part, or both the first part and the second part through one or more bearings or bushings. Bearings are shown by way of example at 572, 574 in Figure 5B.

The movement may also or instead include linear movement generated by the power tool, in which case the movable component may be coupled to the first part, the second part, or both the first part and the second part through one or more bearings, bushings, or diaphragms.

Other types or forms of movement include, for example, rotational oscillatory movement and linear oscillatory movement, either or both of which may be generated by a power tool.

In some embodiments, the movement generated by a power tool is or includes one type of movement, and a transmission component includes a mechanism to convert the one type of movement into another type of movement.

In some embodiments a pass-through such as 16, 416A, 416B is supported by, or otherwise used in conjunction with, an adapter. An adapter may be considered to support a pass-through in the sense that an adapter may provide more physical support to a pass-through than a cover might provide on its own. A cover may be made from or include flexible material(s) for example, and an adapter could be made from or include less flexible or more rigid material(s) to support a pass-through. More generally, such an adapter may adapt a sterile barrier or cover to a transmission component such as a pass-through in some embodiments.

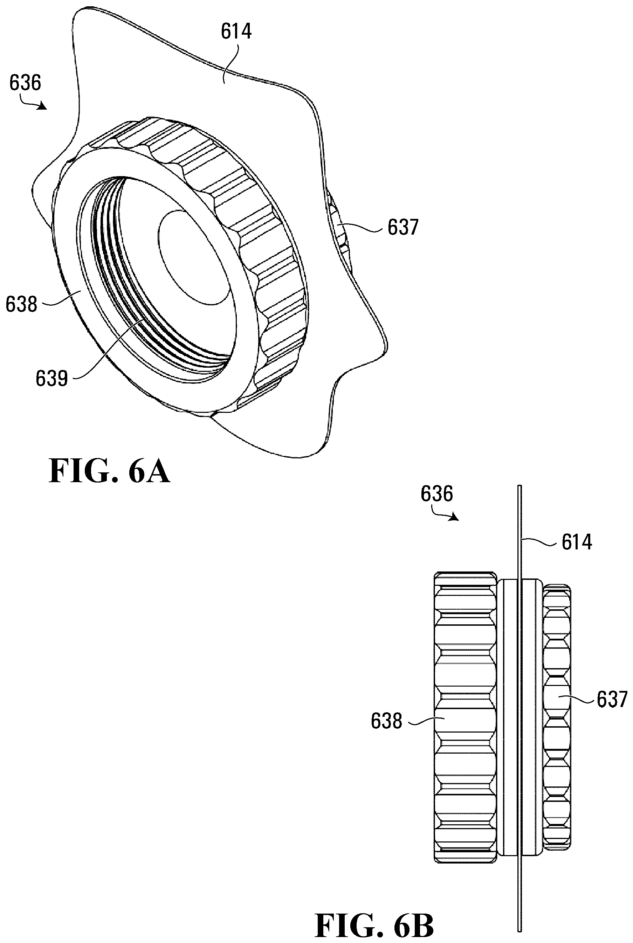

As shown in Figures 6A and 6B, in an embodiment an adapter 636 comprises a nose 638 and a nose mate 637. The nose 638 and the nose mate 637 are coupled together in the views shown in Figures 6A and 6B. A cover 614 may be trapped between the nose mate 637 and the nose 638, thereby forming a seal around an aperture in the cover. The nose mate 637 may be positioned inside an inner cavity of the cover 614, for example prior to inserting a power tool into the inner cavity, and the nose 638 may be outside the cover 614.

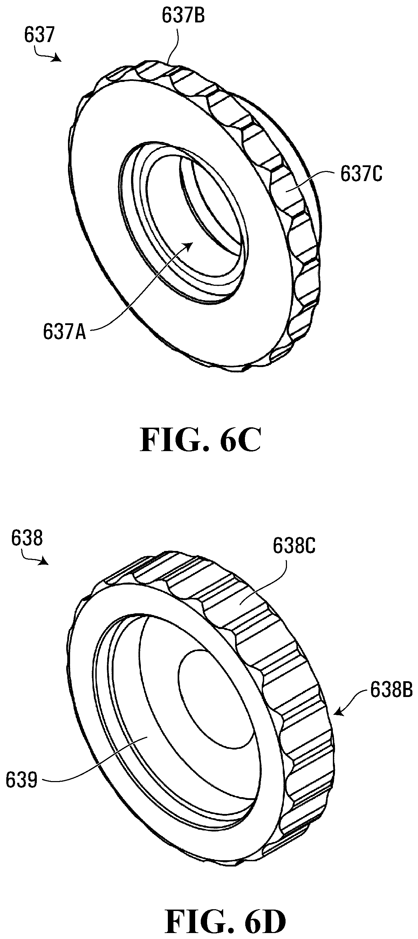

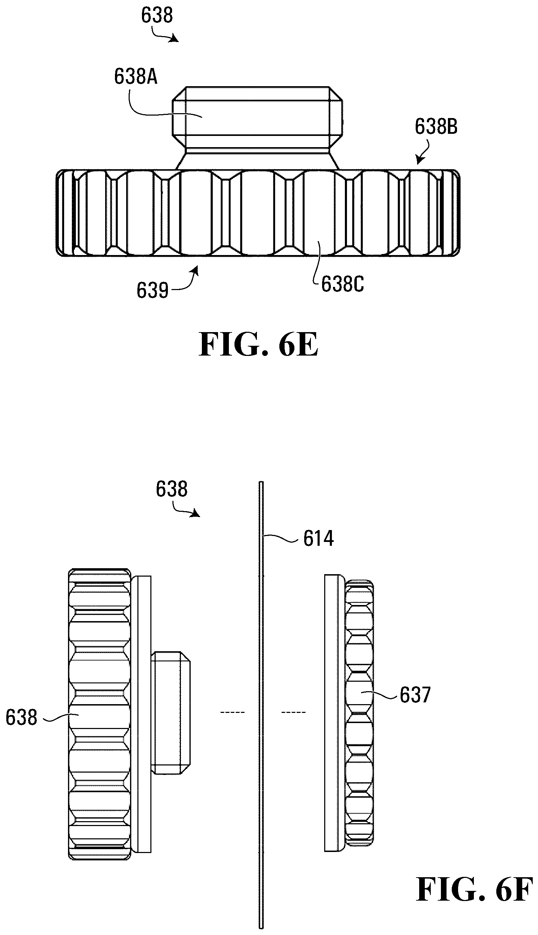

The nose mate 637 may comprise a threaded bore 637A, as shown by way of example in Figure 6C. The nose 638 may comprise a corresponding threaded tubular shaft 638A, as shown by way of example in Figures 6D and 6E. Inserting the shaft 638A into the threaded bore 637A and rotating the nose 638 relative to the nose mate 637 may couple the nose mate 637 with the nose 638. The bore 637A and the shaft 638A may be threaded to position opposing surfaces 637B, 638B of the nose mate 637 and the nose 638, respectively, a sufficient distance apart, such as the distance d referenced elsewhere herein, to form a seal around the aperture in the cover 614. Either or both of the nose mate 637 and the nose 638 may comprise ridges around outer rims 637C, 638C of the nose mate 637 and the nose 638, respectively, to assist with coupling the nose mate 637 to the nose 638. Arrangement of the parts of an adapter relative to a cover for assembly of the adapter is illustrated by way of example in the exploded view shown in Figure 6F.

Although the nose mate 637 and the nose 638 have been illustrated as having a threaded coupling, this is not necessary. The nose mate 637 and the nose 638 may be coupled together using one or more of: a press fit, a magnetic coupling, fasteners, a bayonet coupling, a clip retention coupling, a spring-loaded ball coupling, a spring-loaded bar coupling, a spring-loaded pin coupling, and a spring retention coupling, and the like.

A pass-through such as 16, 416A, 416B may extend through the nose mate 637 and the nose 638. Coupling a pass-through the nose mate 637 and the nose 638 completes a seal of the aperture in the cover 614. The nose 638 may comprise a threaded collar 639. A corresponding threaded portion of a pass-through, such as a threaded bearing assembly configured to support a shaft of the pass-through that is to be coupled to a power tool inside the cover 614, may be coupled with threaded collar 639. Fully engaging the threads of the pass-through with the corresponding threads of threaded collar 639 in this example may alert a user that the pass-through has been inserted fully into the adapter 636.

In some embodiments a gasket may be bonded to or otherwise provided on one or both of the opposing surfaces 637B and 638B of the nose mate 637 and the nose 638, respectively. Advantageously, this may allow the gasket(s) to be reusable, thereby potentially reducing manufacturing costs of the cover 614. In such embodiments, the cover 614 need not comprise a gasket. In some embodiments, the gasket(s) bonded to or otherwise provided on one or both of the opposing surfaces 637B and 638B are sterilizable between uses, for applications in which sterility of the nose mate 637 and the nose 638 is required or desirable.

A pass-through 16 is preferably sterile. In some embodiments, a pass-through may be reused and sterilized a plurality of times. However, in other embodiments a pass-through is a single use disposable component of a power tool system.

Figures 6A to 6E illustrate one embodiment of an adapter. The adapter 636 is representative of an adapter to adapt a sterile barrier to a transmission component, such as a pass-through 16, 416A, 416B, that is configured to transmit movement from a power tool through the sterile barrier. The nose 638 and the nose mate 637 are illustrative of a first part of an adapter, that is to be positioned at a first side of the sterile barrier, and a second part of an adapter, that is to be positioned at a second side of the sterile barrier opposite the first side.

The second part of an adapter is couplable with the first part to trap and compress a portion of the sterile barrier between the first part and the second part and form a compression seal around an aperture in the sterile barrier, as shown perhaps most clearly in Figures 6A and 6B.

One or both of the first part and the second part of an adapter may include a tubular shaft to extend through the aperture and couple the second part with the first part. Such tubular shafts are visible at 637A in Figure 6C and at 638A in Figure 6E, for example.

The first part of an adapter may include or provide a first surface and the second part may similarly may include or provide a second surface, to engage and compress the portion of the sterile barrier at the first side and the second side, respectively. The surfaces 637B, 638B in Figures 6D and 6E are illustrative examples of such surfaces.

An adapter may include one or both of: a compressible gasket on the first surface, and a compressible gasket on the second surface. Such gasket(s) may be provided separately from the adapter. For example, the portion of the sterile barrier that is to be trapped between the first part and the second part of an adapter may include one or more compressible gaskets or have one or more compressible gaskets attached thereto.

The first part and the second part of an adapter may be couplable together via any of various types of couplings. For example, the first part and the second part of an adapter may include respective cooperating components of one or more types of couplings, examples of which are provided elsewhere herein.

An adapter may include one or more fasteners to couple the second part with the first part. One or more fasteners may also or instead be used to couple parts of other multi-part components to each other.

As shown by way of example at 637C, 638C in Figures 6C and 6D, one or both of the first part and the second part of an adapter may include an outer rim with ridges to assist with coupling the second part with the first part. For a re-usable adapter, ridges or other structures may also or instead assist with decoupling the second part and the first part from each other.

An adapter may include a coupling structure to couple with the transmission component. For example, either or both of the first part and the second part of an adapter may include a coupling structure to couple with the transmission component. The threaded collar 639 in Figure 6D is an illustrative example of such a coupling structure.

Many of the embodiments herein refer to an aperture in a cover or sterile barrier, through which power or movement generated by a power tool can be transmitted. Although such an aperture may be formed in or otherwise defined by a cover or sterile barrier, in other embodiments a multi-part device may create the aperture in a cover or sterile barrier. This may provide greater flexibility or applicability of a cover or sterile barrier, in that an aperture may be created at any convenient position or location on the cover or sterile barrier. With the ability to create an aperture, a sterile barrier need not even be intended for use as a cover. This may enable medical staff to use any sterile material that is at hand, such as a sterile glove or gown for example, as a sterile barrier for a power tool.

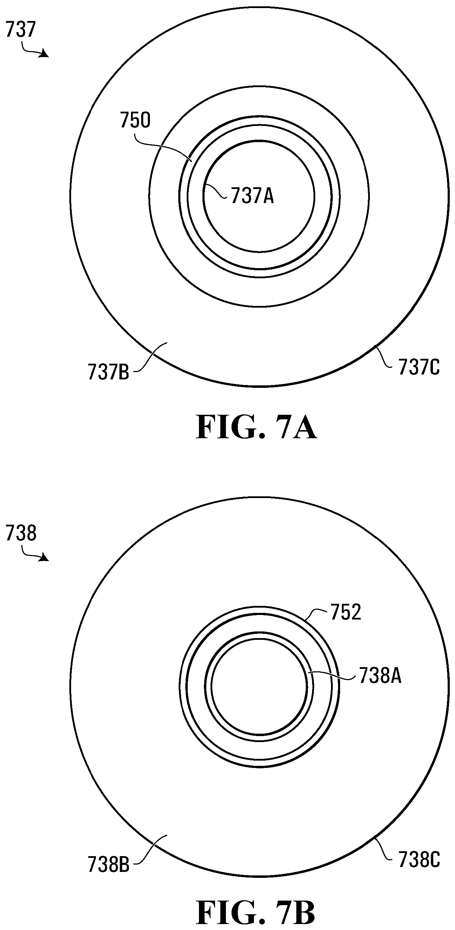

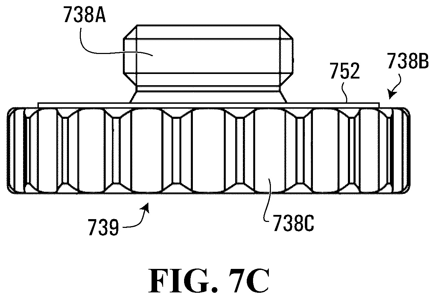

Consider, for example, Figures 7A to 7C. Figure 7A is a top view of part of a multi-part device according to another embodiment, and Figures 7B and 7C are a top view and a side plan view, respectively, of another part of a multi-part device.

Figure 7A illustrates an example of a first part 737 of a multi-part device. The first part 737 is to be positioned at a first side of a sterile barrier. Figures 7B and 7C illustrate a second part 738 of a multi-part device. The second part 738 to be positioned at a second side of the sterile barrier opposite the first side. The second part 738 is couplable with the first part 737 to create an aperture in the sterile barrier and to trap a portion of the sterile barrier between the first part and the second part and form a seal around the aperture. As an example, an outside surface of a column 738A that extends axially from the surface 738B of the second part 738 and an inside surface of a column 737A that extends axially from the surface 737B of the first part 737 could be threaded to couple the first and second parts together.

One or both of the first part and the second part may include a cutter to create the aperture. As an example, a cutter 752 may be attached to or otherwise provided on the surface 738B, to create the aperture as the first part 737 and the second part 738 are coupled together. In an embodiment, the cutter 752 creates the aperture by cutting the sterile barrier as the first part and the second part are being coupled together, and a groove 750 is provided at a top surface of the column 737A of the first part 737 to receive at least part of the cutter 752 as the first part and the second part 738 are fully assembled. This may be useful, for example, to enable cutting of the aperture to be completed and a cut part of the sterile barrier to be removed before a portion the sterile is trapped between the first part 737 and the second part 738.

In an embodiment in which the first part and the second part of a multi-part device include respective cooperating components of a threaded coupling, such as the threaded surfaces noted above, the cutter 752 may be configured to create the aperture during relative rotation between the first part and the second part to couple the first part and the second part together. This is just one example, and others are possible.

For example, the cutter and groove described above may be reversed, with a cutter being provided at 750 and a groove being provided at 752.

As another example, the first part of a multi-part device may include a first surface such as the surface 737B and the second part may include a second surface such as the surface 738B, to respectively engage the portion of the sterile barrier at the first side and the second side, with one or both of the first surface and the second surface comprising a protruding element to pierce the sterile barrier and create the aperture. The protruding element may be or include a protruding ring, for example, and may be provided at 750 and/or 752.

In some embodiments, the first part of a multi-part device includes or provides a first surface and the second part includes or provides a second surface, to engage and compress the portion of the sterile barrier at the first side and the second side, respectively. In such embodiments, the seal is a compression seal between the first surface and the second surface. The surfaces 737B, 738B are examples of surfaces that may be compression surfaces in some embodiments.

One or more a compressible gaskets may be provided, on the first surface and/or the second surface for example. Such compressible gasket(s) may also or instead be provided separately, or be attached to or part of the sterile barrier.

A threaded coupling between the first part 737 and the second part 738 is described above as an example. More generally, the first part and the second part of a multi-part device may include respective cooperating components of any of various types of couplings, such as a threaded coupling, a press fit coupling, a magnetic coupling, a bayonet coupling, a clip retention coupling, a spring-loaded ball coupling, a spring-loaded bar coupling, a spring-loaded pin coupling, or a spring retention coupling to couple the first part with the second part. One or more fasteners may also or instead be used to couple the first part with the second part of a multi-part device.

Several components disclosed herein may be implemented as, or as parts of, a multi-part device. For example, the first part and the second part of a multi-part device may be or include parts of a transmission component to transmit movement from a power tool through a sterile barrier. Examples of a multi-part transmission component are provided elsewhere herein, and parts of such a component may include features to enable an aperture to be created in a sterile barrier.

The first part and the second part of a multi-part device may be or include parts of an adapter to adapt the sterile barrier to a transmission component. Examples of a multi-part adapter are provided elsewhere herein, and parts of such an adapter may include features to enable an aperture to be created in a sterile barrier.

The first part of a multi-part device may be or include a tool adapter to be coupled to a power tool and the second part of a multi-part device may be or include a transmission component to be coupled to the tool adapter to transmit movement from the power tool through the sterile barrier. Again, examples of a tool adapter and a transmission component are provided elsewhere herein, and parts of such an adapter and transmission component may include features to enable an aperture to be created in a sterile barrier. With reference to Figure 4B as an example, an aperture may be formed in the cover 414 when the pass-through 416B is coupled to the power tool 412.

A multi-part device may include other features disclosed herein. For example, one or both of the first part 737 and the second part 738 of a multi-part device may include an outer rim 737C, 738C with ridges to assist with coupling the first part and the second part together and/or decoupling the second part and the first part from each other.

In some embodiments, a multi-part device may be partially or fully assembled to create the aperture, and then temporarily disassembled to remove a cut portion of the sterile barrier so that the cut portion does not interfere with operation of a power tool. In other embodiments removal of the cut portion is not necessary and the cut portion does not impede or otherwise interfere with operation of the power tool.

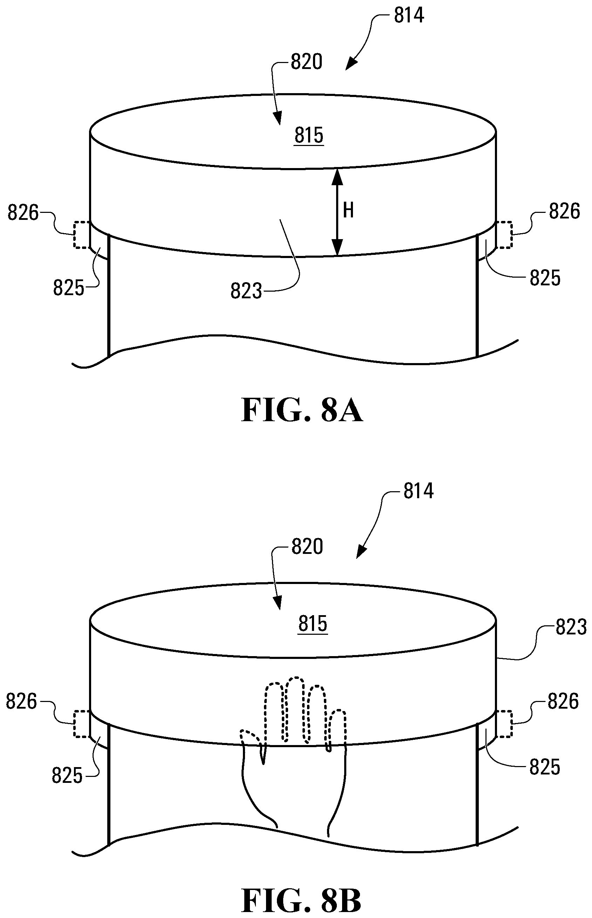

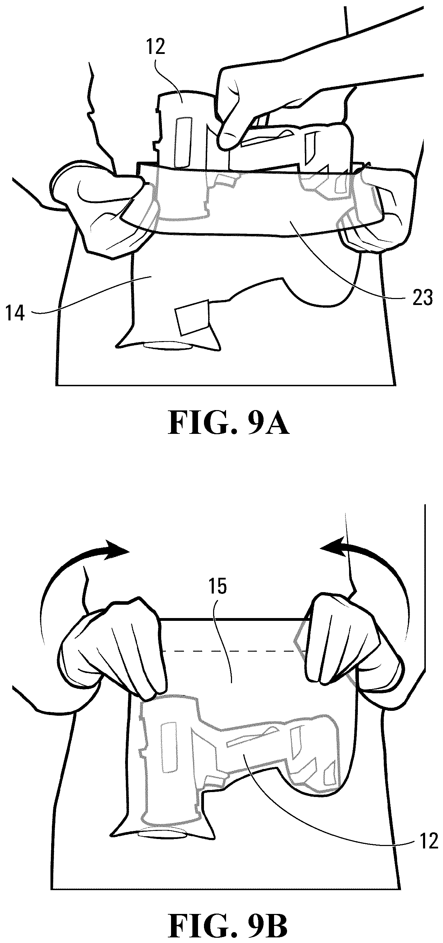

The description above concentrates primarily on structure and components of power tool systems. Other embodiments relate to use of such power tool systems, and are described by way of example with reference to Figures 8A, 8B, and 9A to 9D.

As shown by way of example in Figure 8A, a portion of a cover 814 surrounding an opening 820, through which a power tool is insertable into an inner cavity 815 defined by the cover, is folded over to form a cuff or collar 823 prior to inserting the power tool into the cover 814. The cuff 823 may have a height H which is large enough that the cover 814 may securely be held by placing one's fingers and/or hands behind the cuff 823, between an inner surface of the cuff 823 and an opposing outer surface of the cover 814. This is shown by way of example in Figure 8B. The hand shown in Figure 8B is only a schematic illustration and does not necessarily depict an anatomically correct manner of holding the cover 814. In some embodiments the cuff 823 is at least about 1 inch or about 2.5 cm high. In some embodiments the cuff 823 is at least about 1.5 inches or about 3.8 cm) high. In some embodiments the cuff 823 is greater than 1.5 inches or 3.8 cm high.

Typically, medical professionals scrub-in prior to entering a sterile field such as an operating room. "Scrubbing-in" means antiseptically cleaning ones hands and/or arms prior to entering a sterile field. Once a user has scrubbed in, and typically put on sterile gloves, the cover 814 may be held by the user positioning their fingers and/or hands under the cuff 823 between an outer surface of the cover 814 and an inner surface of the cuff 823 as described elsewhere herein. Doing so neither affects the sterility of the outer surface of the cover 814 nor the sterility of the user's fingers and/or hands. The cuff 823 may also reduce the likelihood of a non-sterile power tool coming into contact with the sterile outer surface of the cover 814.

Once the power tool has been inserted inside cover 814 and into the inner cavity 815, a closing mechanism may be applied or otherwise operated to seal the opening 820. Preferably, the closing mechanism is operable to maintain the opening 820 sealed during ordinary movements and/or uses of a power tool system throughout a surgical procedure. Additionally, the closing mechanism preferably maintains the opening 820 sealed upon the closing mechanism being exposed to a liquid, so that the closing mechanism does not fail upon exposure to a liquid for example.

Figures 9A to 9D are views further illustrating use of a cover system according to an embodiment.

In Figure 9A, the power tool 12 is inserted into the sterile cover 14. The cover 14 has been folded at an edge portion to form a cuff 23, and interaction between a user and the cover 14 using the cuff 23 is perhaps more clearly shown in Figure 9A than in Figure 8B. As shown, a user holds the cover 14 by the cuff 23 while the power tool 12 is being inserted into the cover 14, without comprising a sterile outer surface of cover 14. The cuff 23 reduces the likelihood that the power tool 12, which might not be sterile, will contact a sterile outer surface of the cover 14 as the power tool 12 is being inserted into the cover. The cover 14 may, for example, be held by hands that are sterile, or in other words the person holding the cover 14 has "scrubbed in", while the power tool 12 may be inserted by a person who does not have sterile hands.

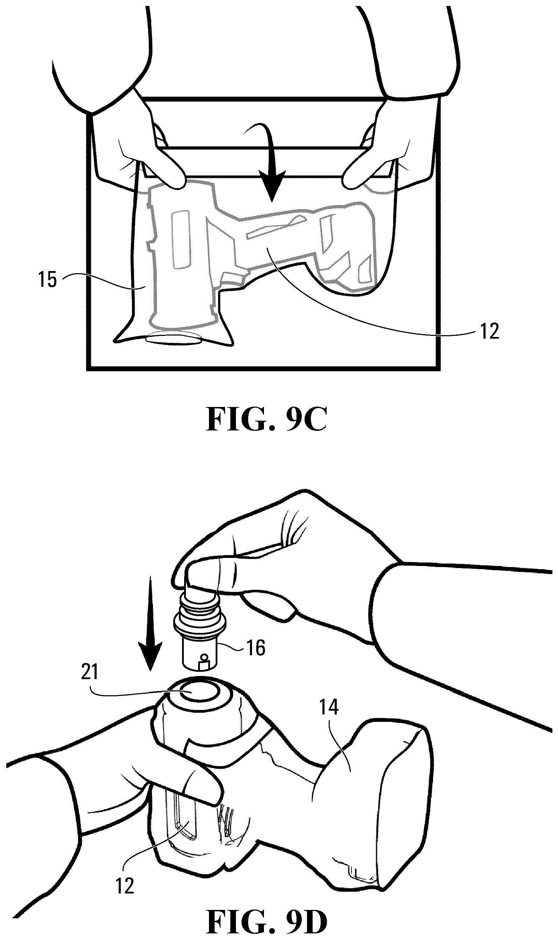

Once the power tool 12 has been inserted into the cover 14, the cuff 23 may be unfolded or unfurled as shown by way of example in Figure 9B, with the power tool inside the inner cavity 15, and the opening through which the power tool was inserted may be closed as shown by way of example in Figure 9C.

The pass-through 16 is coupled to the power tool 12 in Figure 9D, through the aperture 21. In some cases the pass-through 16 is removed from sterile packaging immediately before the pass-through is coupled to the power tool 12. The pass-through 16 may be held with one hand while the other hand is used to orient and hold the power tool 12 relative to the aperture 21 so that the pass-through can be coupled to the power tool through the aperture, by moving the pass-through towards the power tool 12 in the example shown.

The example shown in Figures 9A to 9D is intended solely for illustrative purposes. A method may include additional, fewer, and/or different operations, performed in a similar or different order, depending on one or more factors such as a type of the power tool 12, a type of the pass-through 16, or whether the cover 12 is used with or without an adapter.

In general, a method consistent with the present disclosure may involve orienting an opening of a disposable cover to receive a power tool into an inner cavity defined by the disposable cover that has a sterile outside surface and further defines an aperture. An example is shown in Figure 9A, in which a sterile user is holding the cover 14 with the opening at the top held open to receive the power tool 12.

Such a method may also involve operating a closing mechanism to close the opening with the power tool inside the inner cavity with a drive part of the power tool adjacent the aperture. This is shown by way of example in Figure 9C.

In some embodiments, a method involves forming a compression seal around the aperture to seal the power tool inside the inner cavity. In the example shown in Figure 9D, a compression seal may be formed when the pass-through 16 is coupled to the power tool through the aperture 21. This is an example of forming a compression seal by coupling, to a drive part of a power tool, a transmission component to transmit movement from the power tool through the disposable cover. The drive part of a power tool refers to a part that is driven by the power tool, which in turn drives a transmission component to transmit movement through a cover.

Forming a compressing seal may include compressing one or more compressible gaskets surrounding the aperture, and various examples of compressible gasket arrangements are provided elsewhere herein. One or more compressible gasket(s) may be attached to or part of a cover, or provided separately and placed adjacent to the aperture.

Another embodiment of forming a compression seal involve coupling multiple parts of a multi-part device together to trap a portion of the disposable cover between the parts of the multi-part device. Examples of multi-part devices and how such devices may be coupled together are provided elsewhere herein.

Some embodiments may involve applying pressure to the disposable cover to force air through a one-way valve from the inner cavity to outside the disposable cover.

An aperture through which power or movement may be transmitted through a cover may or may not already be formed or otherwise provided when a cover or sterile barrier is to be used. As such, some embodiments also involve creating the aperture. Forming the aperture may be inherent in other operations, such as coupling parts of a multi-part device together after those parts are placed at opposite sides of a cover or sterile barrier. A multi-part device may be installed in a cover to create the aperture before or after the power tool has been inserted into the inner cavity, for example.

The actions or operations outlined in the example method above relate primarily to actions or operations that may be performed by the sterile user referenced in the description of Figures 9A to 9D. At least some of these operations, and/or others such as placing the power tool inside the inner cavity with the drive part of the power tool adjacent the aperture, may be performed by another user, and potentially a non-sterile user.

Features that may be relevant to these and/or other operations, or to other embodiments, are further discussed below.

With reference now to Figure 2A as an example, the closing mechanism includes an adhesive band 24 which extends across a first portion of the cover 14 and a sealing flap 25 which extends across a second portion of the cover 14. The sealing flap 25 is a longitudinally extending sealing flap and the adhesive band 24 is a longitudinally extending adhesive band in the example shown, but other orientations or arrangements are also possible.

Engaging the sealing flap 25 with the adhesive band 24 bonds the sealing flap 25 to the cover 14, thereby sealing the opening 20 closed. The sealing flap 25 may, for example, be folded over lengthwise for the sealing flap 25 to engage the adhesive band 24. Preferably, the adhesive band 24 is covered by a removable non-adhesive layer 24A until the opening 20 is to be closed. For example, the removable non-adhesive layer 24A may be removed after a power tool has been inserted into the cover 14.

The adhesive band 24 may comprise, for example, one or more of: a suitable adhesive, double sided tape, and the like. In preferred embodiments, exposure of the adhesive band 24 and the sealing flap 25 to a liquid does not un-bond the sealing flap 25 from the adhesive band 24.

Strength of the bond between the sealing flap 25 and the cover 14 may be increased by having a plurality of bonds between the adhesive band 24 and the sealing flap 25. For example, the adhesive band 24 may have a width which is greater than a width of the sealing flap 25. In such embodiments the sealing flap 25 may be folded over lengthwise a number of times so that it engages the adhesive band 24 a number of times, for example until all of the adhesive band 24 has been engaged. In some embodiments, the adhesive band 24 has a width that is at least double the width of the sealing flap 25. Folding the sealing flap 25 over a number of times may also lower the likelihood of a liquid being able to penetrate through the opening 20 after the opening has been closed.

Although the sealing flap 25 and the adhesive band 24 have been shown in Figure 2A as having a uniform width, this is not mandatory. In some embodiments a width of particular portions of the adhesive band 24 and/or the sealing flap 25 may be increased relative to other portions of the adhesive band 24 and/or the sealing flap 25. For example, portions of the adhesive band 24 and/or the sealing flap 25 which may have a higher likelihood of failure, by separating apart from one another for example, may have increased width relative to the remaining portions of the adhesive band 24 and/or the sealing flap 25.

As described elsewhere herein, a cover such as the cover 14 may be a disposable sterile cover that is intended to be used for a single surgical procedure and/or a single patient. To discourage or prevent reuse of the cover 14 for multiple surgical procedures having different patients, the bond between the sealing flap 25 and the adhesive band 24 may be designed to be strong enough that removal of a power tool from the cover 14 requires at least partial destruction of the cover 14. Once at least a portion of the cover 14 is destroyed, the cover 14 can no longer be used and a new cover 14 must be obtained. In this sense, a closing mechanism may be arranged, designed, or otherwise configured to permanently close the opening 20. For example, the bond between the sealing flap 25 and the adhesive band 24 may be strong enough such that removal of a power tool from the cover 14 requires cutting the cover 14 open, with scissors for example, tearing the cover 14 open, or otherwise damaging the cover 14 in such a way that is it not re-usable. However, this is not mandatory. In some embodiments the sealing flap 25 may be peeled away from the adhesive band 24, for example, to correct a position of the sealing flap 25 relative to the adhesive band 24.

Optional tabs 26 may assist a user with folding over the sealing flap 25 while reducing the likelihood of the user having a finger or fingers trapped between the sealing flap 25 and the adhesive band 24. One or more tabs may be provided, and arranged along an edge of the sealing flap as shown. Such a tab is operable to facilitate folding of the sealing flap 25 towards the adhesive band 24.

The example cover 14 includes a tab 26 on each opposing longitudinal end of the sealing flap 25. A user may hold each tab 26 in a different hand. Pulling each tab 26 toward the adhesive band 24 folds the sealing flap 25 over toward the adhesive band 24. However, the cover 14 may comprise any number of tabs 26, positioned anywhere along the sealing flap 25. In some embodiments the cover 14 comprises a single large tab 26 along an upper edge of the sealing flap 25 in the view shown in Figure 2A. In such embodiments the sealing flap 25 can be folded over by pulling tab 26 towards an opposing end of the cover 14.

In some embodiments either or both of the sealing flap 25 and the adhesive band 24 are replaced with or augmented by an alternative closing mechanism such as:
- a tongue and groove closure similar to a ZIP-LOC^{™} type closure;
- a hook and loop closure such as a VELCRO^{™} type closure;
- a pull strap;
- one or more snaps;
- a magnetic seal;
- a belt and buckle design; and
- a zipper seal.

These are illustrative examples of a closing mechanism, any one of which may be attached to a cover in some embodiments. One or more closing mechanisms may be integrated with or be part of a cover, or a cover may otherwise include one or more closing mechanisms.

The cover 14 may be shaped to conform generally with the shape of a power tool. For example, where the power tool is a drill, power screwdriver, impact driver, or other tool having a handle that extends generally at right angles to a body, then the cover 14 may have a generally L-shaped configuration as shown in Figure 2A. With this type of power tool and cover configuration, the body of the power tool fits into one arm of the L-shaped cover and the tool handle fits into the other arm of the L-shaped cover. In some embodiments an arm of the cover 14 which receives the body of a power tool extends generally perpendicular to the opening 20 such that when the cover 14 is held with the opening 20 uppermost and open, the arm of the cover 14 which receives the body of the power tool extends generally vertically.

The cover 14 may be sized to receive a power tool. Different sizes of the cover 14 may be provided for use with different power tools. In some embodiments the cover 14 is approximately 30 cm x 25 cm when laid flat as shown in Figure 2A.

Preferably the cover 14 is fitted to a shape of a power tool. Excess space between the cover 14 and the power tool may reduce tactility, thereby reducing a user's ability to control the power tool. For example, excess material of the cover 14 may impede a user's ability to precisely control a trigger of the power tool. The cover 14 may comprise one or more straps configured to allow a user to fit the cover 14 to the shape of particular power tool that is placed into the inner cavity of the cover 14.

One or more straps 27 may, for example, be provided. Such strap(s) may be used to collect excess portions of the cover 14 and/or to shape the cover 14 to a desired shape. As shown in Figure 2A, for example, the cover 14 may comprise two straps 27. A strap 27A may be proximate to a portion of the cover 14 which is intended to receive the handle of a power tool and a strap 27B may be proximate a portion of the cover 14 which is intended to receive the body of the power tool.

The straps 27 are attached to the cover 14 in the example shown. However, each strap 27 comprises a detachable end which may be detached from the cover 14. The detachable end of each strap 27 may initially be attached to the cover 14. Detaching a detachable end of a strap 27, repositioning and/or wrapping the strap generally closely or tightly around part of a power tool, and then re-attaching the detached end to the cover 14 may fit a portion of the cover 14 to the part of the power tool. This is perhaps best shown in Figure 1A, in which the straps 27 have been wrapped around parts of the power tool 12 to fit respective portions of the cover to a shape of the power tool. In this manner the straps 27 are operable to shape at least a portion of the cover to a shape of the power tool.

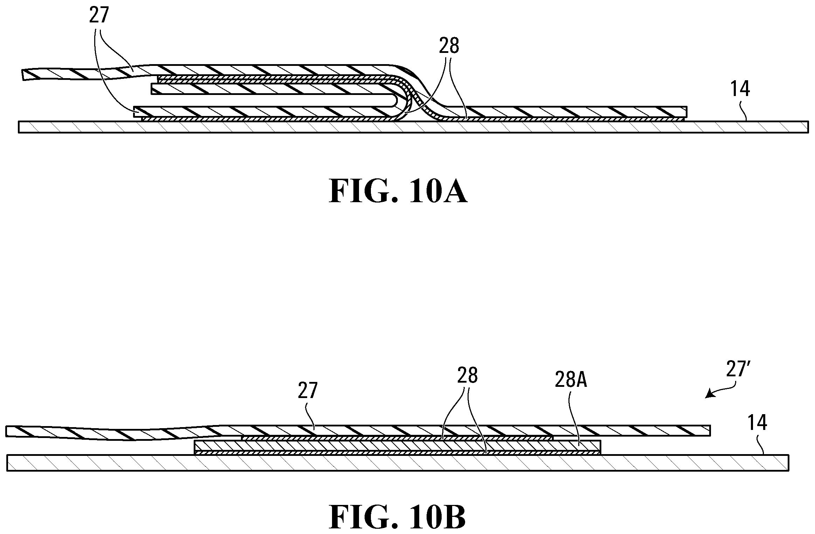

The straps 27 may be bonded to the cover 14. For example, with reference to Figures 10A and 10B, an adhesive layer 28 may bond the straps 27 to the cover 14. The adhesive layer 28 may comprise one or more of: a suitable adhesive, double sided tape, and the like. In some embodiments the adhesive layer 28 is replaced with a heat weld or the like. In Figure 10A, the straps 27 are bonded to each other, but this is to illustrate how the straps may be arranged during packaging or shipping, or otherwise when a cover is not in use. The straps 27 may be detached at least from each other, and potentially entirely detached from the cover, and repositioned as needed or desired to better fit one or more parts of the cover to one or more parts of a tool.

Detachable ends 27' of the straps 27 may also comprise adhesive layer 28. In such embodiments, the detachable ends 27' may initially be attached to a non-adhesive backing layer 28A, as shown by way of example in Figure 10B, and later removed and repositioned to better fit one or more parts of the cover to one or more parts of a tool. Shipping the cover 14 with the detachable end 27' of each strap 27 attached to a non-adhesive backing layer 28A, or to each other as shown in Figure 10A, may reduce the likelihood of a detachable end 27' of a strap 27 bonding to the cover 14, and may also or instead reduce the likelihood of punctures or tears that may be caused as a result of a detachable end 27' having to be detached from the cover 14 directly.

The straps 27 represent one example of a cover fitting mechanism that is operable to fit a portion of the cover to a shape of the power tool. Such a cover fitting mechanism may be attached to the cover. A cover fitting mechanism may be integrated with or part of the cover, or the cover may otherwise include the cover fitting mechanism. A strap is an example of a cover fitting mechanism, and may include a detachable end operable to be detached from the cover, wrapped around the portion of the cover and reattached to the cover to fit the portion of the cover to the shape of the power tool. The detachable end of a strap may include an adhesive layer or otherwise be attached to the cover by a non-adhesive backing layer.

To avoid tears or punctures, the cover 14 may be made of a puncture and tear resistant material. For example, the material may use one or more standards such as EN 388:2016 and AAMI PB70 as guidance. Having a puncture or tear in the cover 14 would breach the sterile barrier that is intended to be provided by the cover 14. Either or both of puncture resistance and tear resistance may be quantified using an industry standard, such as ASTM F1342 Standard Test Method for Protective Clothing Material Resistance to Puncture. Ideally, the cover 14 is classifiable or qualifies as puncture proof, tear proof, or both. In some embodiments the cover 14 has the same or a greater puncture resistance and/or tear resistance as sterile gloves worn by medical professionals.

Preferably the cover 14 is stretchable enough to allow for stretching of the cover 14 to eat least some degree upon the cover 14 being impacted. The cover 14 need not necessarily be resilient. For example, the cover 14 need not necessarily return to its original state after impact, or upon a power tool being removed from the cover.

The cover 14 may, for example, be made of or include any one or more of the following materials:
- Polyethylene (PE) film (preferred);
- Polyurethane (PU) film;
- nonwovens, such as spunlaced, spunlaid heat bonded, meltblown, spunbond, airlaid, or combinations thereof such as Spunbond Meltblown Spunbond (SMS);
- silicone; and
- rigid plastics.

In some embodiments materials forming the cover 14, such as a PE film, are thin and have thicknesses of less than about 0.3 mm. In some embodiments a thickness of the cover 14 is in the range of 0.1 mm to 0.2 mm, such as 0.15 mm.

Increasing the strength of the cover 14 may improve puncture and/or tear resistance of the cover 14. Strength may be increased by, for example, making the cover 14 from a thicker material, a harder material such as a more rigid material, or a stronger material, for example. This may, however, increase a cost of the cover 14. In addition, increasing strength of the cover 14 may reduce or otherwise affect one or more characteristics of the cover, such as flexibility and/or tactility of the cover 14.

Flexibility and/or tactility of the cover 14 is important. With reference to Figure 1A, cover flexibility and/or tactility may be important because a user needs to be able to grip the power tool 12 through the cover 14 and to precisely position the power tool 12 and operate a trigger of the power tool 12 through the cover 14. Flexibility and/or tactility of the cover 14 may be increased by making the cover 14 thinner, more flexible, and/or from a softer material, for example. Increased flexibility and/or tactility of the cover 14 may improve a user's ability to grip and operate the power tool 12.

Improving puncture and/or tear resistance of the cover 14 generally conflicts with a desire to improve flexibility and/or tactility of the cover 14. In preferred embodiments a desirable puncture and/or tear resistance is balanced against a desirable flexibility and/or tactility of the cover 14.

In some embodiments different portions of the cover 14 are made of different materials. For example, portions of the cover 14 that are intended to surround a grip and/or trigger of the power tool 12 may be made of a flexible and tactile material. In an embodiment, a trigger portion of the cover 14 surrounding a trigger area of the power tool, or that is to be positioned adjacent to a trigger area of the power tool, is more tactile than another portion of the cover. Remaining portions may be made of a stronger material that is less flexible and tactile but more puncture and/or tear resistant.

Additionally, or alternatively, a thickness of the cover 14 may be increased in portions of the cover 14 that are expected to have a higher risk of a puncture and/or tear. In some embodiments such portions comprise two or more layers of materials. Each layer of material may be the same as or different from other layers. In some embodiments the increased thickness dampens and/or absorbs impacts of objects coming into contact with the power tool system 10. In some embodiments the portions having increased thickness act as bumpers.

In some embodiments the cover 14 or select portions of the cover 14 may comprise the multi-layer construction. In some embodiments the multi-layer construction spans the cover 14 in its entirety, and the cover comprises multiple layers. In some embodiments the inside surface of the cover 14 is made of a first layer and the outside surface of the cover 14 is made of a second layer. The first and second layers may be bonded together. The first and second layers may be the same or different, and accordingly the inside of the cover 14 may be or include a different layer than the outside of the cover. In some embodiments the first and second layers have one or more different properties, such as different colours and/or different opacities. **In** some embodiments the first and second layers have the same colours, opacities, and/or one or more other common properties that are the same. Two or more different portions of the cover may comprise different numbers of layers in some embodiments.

Referring again to Figure 2A, in some embodiments a multi-layered construction of the cover 14 creates a tortuous path at the opening 20 and/or the aperture 21, reducing the likelihood of the sterile barrier being breached. For example, the sealing flap 25 may comprise multiple layers that may be folded over and engaged with the adhesive band 24. In some embodiments, multiple layers of the cover 14 may be trapped between compression members, such as the compression members 317A, 317B shown in Figures 3A to 3E. A liquid, for example, would need to pass or penetrate through each layer prior to entering or leaving the inner cavity of a cover in such embodiments.

Additionally, or alternatively, a stronger secondary cover may be placed over portions of a cover that are expected to have a higher risk of a puncture and/or tear, such as a higher likelihood of coming into contact with a scalpel or other tool. For example, a sterile secondary cover 18 is shown by way of example in Figure 1A, and may be placed over an upper portion of the cover 14.

The secondary cover 18 comprises an aperture 18A. Pass-through 16 may extend through aperture 18A. An opposing end 18B of the secondary cover 18 may be stretched over the body of the power tool 12 and may engage an opposing end of the power tool 12 as shown in Figure 1A.

The secondary cover 18 may be made of or include one or more materials such as silicone, rubber, and vinyl. In some embodiments the secondary cover 18 comprises a hard shell configured to fit over a portion of the power tool system 10. The secondary cover 18 may be disposable or sterilizable and reusable.

Optionally, the cover 14 may comprise one or more shaped corners, shown by way of example at 29 in Figure 2A. Such a shaped corner 29 may provide more space within an inner cavity of the cover 14 than a corner comprising edges that converge at a vertex. This may, for example, reduce strain on seams of the cover 14 upon a power tool being inserted into the cover 14, and/or may provide more space for a trigger of a power tool 12 to travel. The shaped corner 29 may be any shape other than a point or a vertex. The shaped corner 29 may, for example, be circular, ellipsoidal, or linear. In some embodiments the cover 14 comprises at least one shaped corner 29 proximate to a trigger area that is to be positioned adjacent a trigger of a power tool.

Inside and/or outside surfaces of the cover 14 may have any of various particular finishes. Finishes of the inside and outside surfaces may be the same or different.

An inside surface of the cover 14 may, for example, be one or more of non-linting and non-static. Preferably the inside surface is finished in a manner that allows for easy insertion of a power tool, such that the inside surface does not impede insertion of a power tool for example.

An outside surface of the cover 14 may also, for example, be one or more of non-linting and non-static. Additionally, or alternatively, the outside surface may be designed to reduce sticking of the outside surface to itself. Additionally, or alternatively, the outside surface, or a gripping portion of the outside surface for example, may be designed not to become slippery to hold when the cover 14 is covered in a liquid such as blood or saline. In some embodiments the outside surface, or a gripping portion of the outside surface for example, may be designed to absorb liquids to increase grip, such as to make the cover 14 less slippery to hold in the presence of a liquid. In some embodiments a biocompatible moisture wicking coating is applied to the outer surface.

Inside and/or outside surfaces of the cover 14 which are anti-static may advantageously reduce the likelihood of particulates such as contaminants becoming electrostatically attracted to the cover 14.

In some embodiments an antibacterial coating is applied to the inside and/or outside surface of the cover 14, or one or both of the surfaces otherwise comprise an antibacterial coating.

In some embodiments the cover 14 is constructed using a pattern, such as a two dimensional pattern. The pattern may be cut from a piece of material using a die, for example. The pattern may be folded into a desired shape of the cover 14. Edges coming into contact with one another may be welded, bonded, sewn, or otherwise joined together. In some embodiments the pattern is folded such that the portion of the cover 14 that includes the aperture 21 and the gasket(s) 22 is flat in the folded cover 14. This may avoid creases in the cover 14 in the area of the aperture 21.

In some embodiments the cover 14 is manufactured using a molding process. For example, the cover 14 may be manufactured by dip-molding, injection molding or the like. The molding process may use an elastomeric compound.

Preferably seams of the cover 14 are designed to be narrow. In some embodiments seams of the cover 14 have a width of 2 mm or less, such as 1.5 mm. In some embodiments seams cross a trigger area of a power tool. Having wide seems may impede tactility of the trigger area.

A power tool is preferably inserted into or otherwise enters the cover 12 from the back of cover, for example through the opening 20 as shown in Figure 2A. However, a power tool may instead be inserted into or otherwise enter a cover from a bottom or top of the cover in some embodiments.

The cover 14 may be designed to withstand a range of temperatures. For example, the cover 14 may be designed to be useable in ambient temperature ranges expected in operating rooms. In some embodiments the cover 14 is designed to be usable in temperatures between 0°C and 40°C.

In some embodiments air may be removed from inside the cover 14 to better fit the cover 14 to a power tool. For example, a mechanism such as a vacuum may remove air prior to sealing the opening 20 and/or the aperture 21 of the cover 14. In some embodiments the cover 14 comprises a one-way valve 40 which allows air to be squeezed out from inside the cover 14. In some embodiments the one-way valve 40 comprises a filter which prevents matter such as particles, spores, bacteria, or molds, from escaping the inner cavity of the cover 14. The one-way valve 40 is preferably located at a position where it is not expected to interfere with operation of a power tool inside the cover 14. A one-way valve may be attached to the cover 14. A one-way valve may be integrated with or part of the cover 14, or the cover may otherwise include such a valve.

In some embodiments the cover 14 comprises markings. For example, the removable non-adhesive layer 24A which covers the adhesive band 24 may comprise markings which illustrate how the cover 14 is to be closed. As another example, the cover 14 may comprise branding. As another example, the cover 14 may comprise markings which instruct a user or describe how to operate a power tool. Markings may be printed directly onto the cover 14, for example.

In some embodiments the cover 14 comprises a rigid or hard portion. In such embodiments portions of the cover 14 which are to surround or be positioned adjacent to a trigger area of a power tool may be made of a more flexible material to potentially allow for more precise operation of the power tool trigger.

Preferably, the cover 14 may be packaged compactly and/or flatly. This may reduce a spatial footprint of the cover 14, how much space is required to store the cover 14, and/or transport costs, for example.

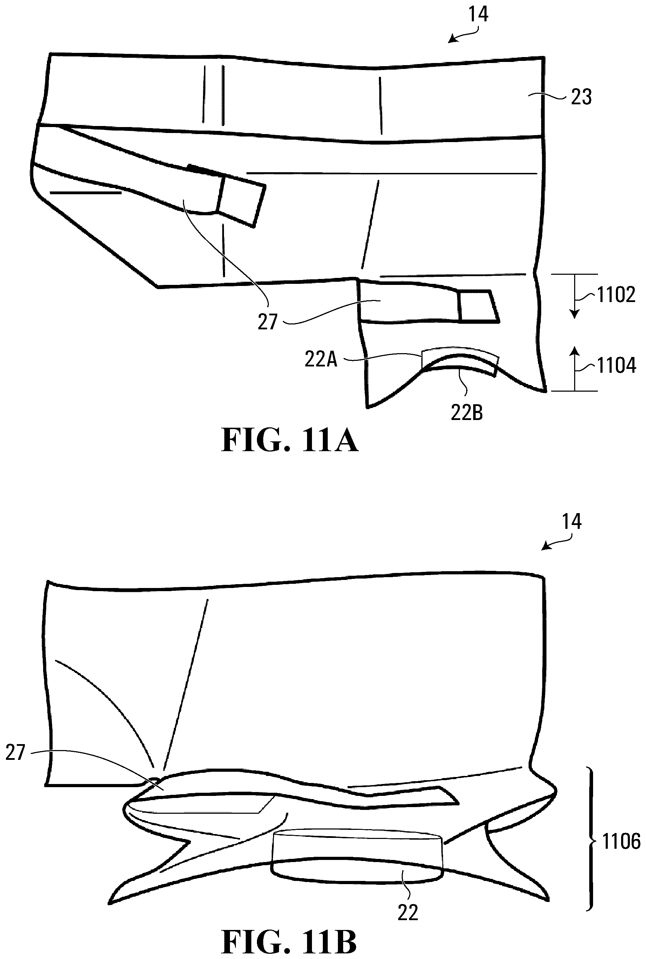

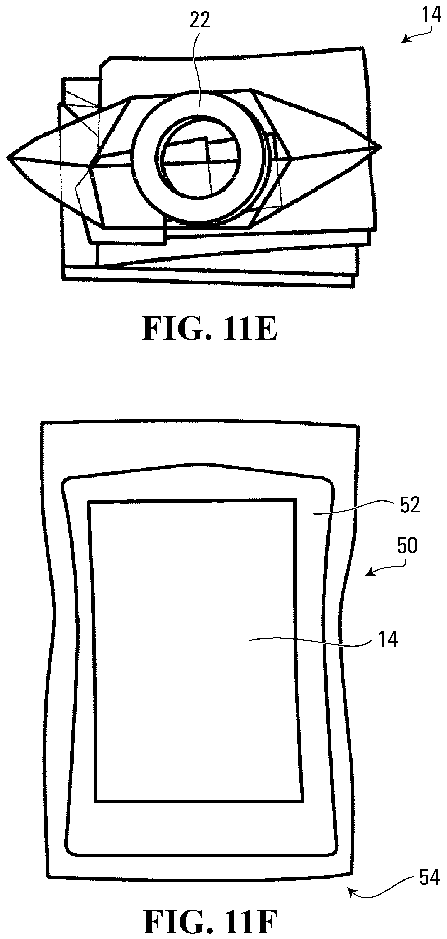

The cover 14 may be folded flat into compact packaging 50 as shown in Figure 11F. Figures 11A to 11E illustrate example folding patterns that may be used to, for example, fold the cover 14 into compact and flat packaging 50.

In Figure 11A portions of the cover 14 which surround the opening 20 are at the top of the illustrated view, and are optionally folded over to form a cuff 23. In such cases, the cover 14 is packaged with the cuff 23pre-formed. This advantageously may reduce time that is required to prepare a power tool system prior to a surgical procedure, in that additional time does not have to be spent to form the cuff 23.

In Figure 11B, side walls of a portion of the cover 14 proximate to an aperture and gaskets 22A and 22B are folded, at least partially inward for example, to form accordion folds. The accordion folds may allow that portion of the cover 14 to be folded flat, as illustrated at 1102, 1104 in Figure 11A, relative to the remaining portions of the cover 14. Folding that portion flat may reduce the likelihood of damaging gaskets 22A, 22B while the cover 14 is being stored and/or transported, for example. An accordion fold is generally denoted by 1106 in Figure 11B.

Additionally, or alternatively, accordion folds may be persistent. "Persistent" means that inserting a power tool inside the cover 14 does not fully destroy the accordion folds. The persistent accordion folds may, for example, take up any slack in the cover 14 when a power tool is inserted inside the cover 14.

Additionally, or alternatively, the accordion folds may assist with conforming the cover 14 to a shape of a power tool. The accordion folds may for example decrease a length of a portion of the cover 14 which receives the body of a power tool. The accordion folds may, for example, be extended only to a length necessary to fit a body of a power tool. Power tools having longer bodies may extend more accordion folds than tools having shorter bodies.

In some embodiments the side walls are folded inward along a single point to form a single accordion fold on each side. In some embodiments the side walls may be folded inwards along two or more points to form two or more accordion folds on each side.

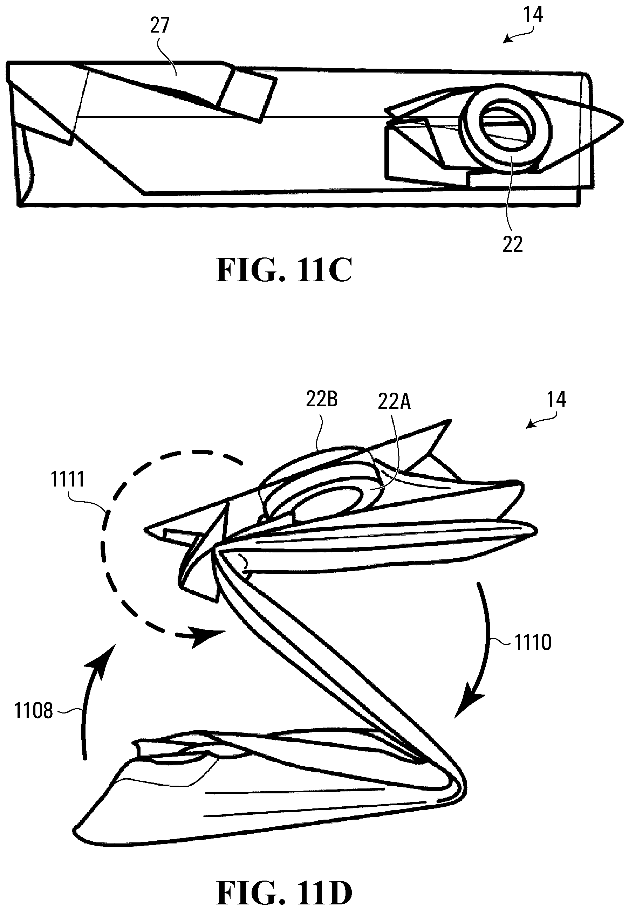

Figures 11C and 11D illustrate example steps to reduce a spatial footprint of the folded cover. In Figure 11C, the cover 14 is folded lengthwise a number of times to reduce an overall width of the folded cover. In Figure 11D, the cover 14 is folded laterally a number of times, as illustrated by the arrows 1108, 1110, to reduce an overall length of the folded cover.

Figure 11E shows a cover 14 that has been folded flat according to a folding pattern illustrated by the arrows 1108, 1110 in Figure 11D. The folded cover has a reduced spatial footprint compared to an unfolded cover 14.

The dashed arrow 1111 in Figure 11D illustrates another example of a folding step that may be performed in some embodiments. According to the example folding step illustrated at 1111, a part of the cover 14 that includes the aperture and the gaskets 22A, 22B is folded in a direction opposite to that shown at 1110 in Figure 11D, and that portion of the cover is positioned between other parts of the cover when the cover is further folded as shown at 1108.

A folded cover, whether folded as illustrated at 1110 or 1111 in Figure 11D for example, may be enclosed in sealed protective packaging 50 as shown in Figure 11F.

Outer surfaces of the packaging 50, such as a top surface 52 and a bottom surface 54 of the packaging 50, may be peeled away from one another to expose the cover 14. This may allow a user to remove the sterile cover 14 from the packaging 50 without the sterile cover 14 coming into contact with one or more non-sterile outer surfaces of the packaging 50. Typically, a non-sterile user would peel the outer surfaces of the packaging 50 while a sterile user, such as a user who has scrubbed in, would remove the sterile cover 14.

The cover 14, and/or any other disposable component described herein, may be sterilized in a manner compatible with the material(s) of the cover 14 or other component. For example, the cover 14 may be sterilized using ETO (ethylene oxide sterilization), radiation, and/or other sterilization processes. In some embodiments the cover 14 may be sterilized after being enclosed by a protective packaging, such as the packaging 50. Reusable components described herein, such as a pass-through 16, 416A, 416B in some embodiments, may be sterilized using steam.

The cover 14 may have a shelf-life of several years. In some embodiments the cover 14 has a shelf-life of 5 years. In some embodiments the cover 14 has a shelf-life of 10 years.

As described elsewhere herein, portions of the cover 14 may be thicker than other portions of the cover 14. In some embodiments one or more gaskets shown by way of example at 22 in Figure 2A are provided by increasing the thickness of the cover 14 around the aperture 21, having multiple layers around the aperture 21 and/or the like. This may have such advantages as simplifying manufacturing of the cover 14 and/or reducing cost, for example.

Embodiments of a power tool system, a cover, a cover system, parts, components, and methods have been described above primarily in the context of surgical applications for human patients. However, the embodiments described herein may also or instead be applied to for veterinary applications, tissue harvesting applications, food processing applications or other applications where enclosing a power tool with a barrier is desirable, such as in an application in which a power tool needs to be protected from its outer environment.

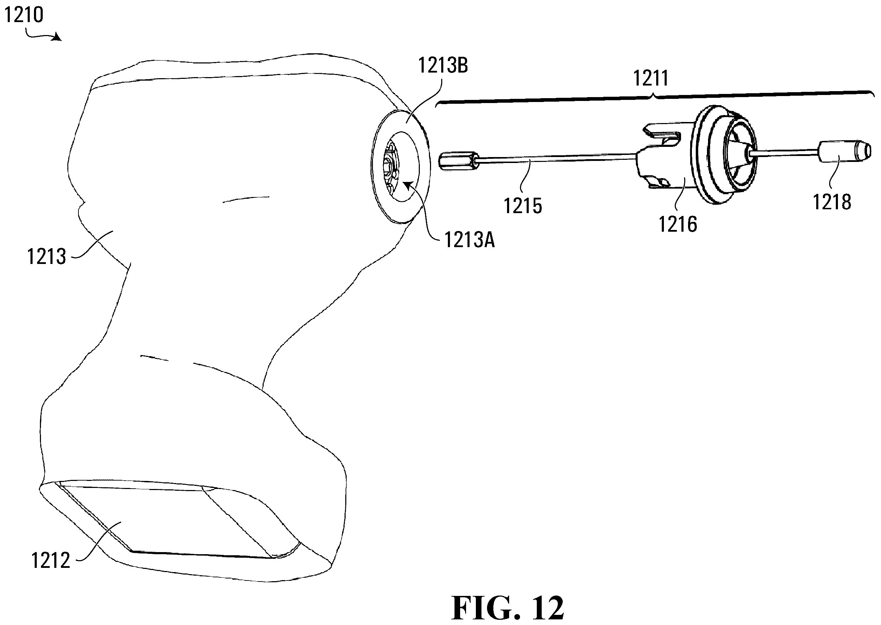

Other variations are also contemplated. For example, Figure 12 is a schematic perspective view of another power tool system 1210, which illustrates a further example of a pass-through. The power tool system 1210 includes a cover 1213 that defines an aperture 1213A, a power tool 12 inside the cover 1213, and an assembly 1211 which is removably couplable to the power tool through the aperture.

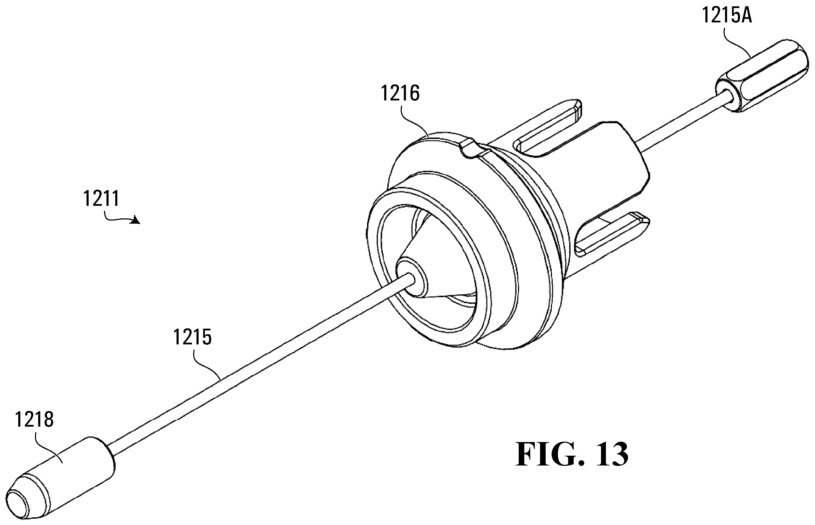

Figure 13 is an enlarged perspective view of the example assembly 1211, which includes a traction pin 1215, an adapter 1216, and an optional cap 1218. The adapter 1216 may be coupled to a corresponding receiving end of the power tool 12 to axially retain the traction pin 1215 relative to the power tool 1212. Coupling the adapter 1216 to the receiving end of the power tool 1212 may also seal the aperture 1213A (Figure 12) through which the traction pin 1215 passes when the power tool system 1210 is assembled. The optional cap 1218 covers a sharp tip of the traction pin 1215 to prevent or at least reduce the likelihood of inadvertent puncturing of tissue or other injury or damage prior to the traction pin 1215 being inserted into a patient's tissue.

Components of the traction pin assembly 1211 are typically sterile. At least some components of the traction pin assembly 1211 may be single-use disposable components and discarded after use on one patient, and the traction pin assembly may also or instead include one or more multi-use components that may be sterilized between uses on different patients.

In some embodiments the traction pin assembly 1211 is packaged in a preassembled state, with the adapter 1216 and cap 1218, if included, already coupled to the traction pin 1215. In some embodiments the traction pin assembly 1211 is packed within sterile packaging.

The adapter 1216 and/or the optional cap 1218 of the traction pin assembly 1211 may, for example, be made of a suitable plastic. In some embodiments such components of the traction pin assembly 1211 are manufactured using such techniques or processes as injection molding, 3D printing, and/or the like.

The adapter 1216 include features to engage corresponding features of the receiving end of the power tool 1212 to couple the adapter to the power tool. The adapter 1216 may help keep an end 1215A of the traction pin 1215 within a coupling mechanism of power tool 1212 while the traction pin is being inserted into tissue of a patient. Once the adapter 1216 is coupled to the receiving end of the power tool 1212, the adapter may become axially fixed and thus might not move axially relative to the power tool until the adapter is decoupled from the power tool. Additionally, the adapter 1216 may seal the aperture 1213A of the sterile cover 1213 through which the traction pin 1215 passes. One or more compressible gaskets 1213B may be provided around the aperture 1213A to potentially improve such sealing. A cover adapter as disclosed by way of example above with reference to Figures 6A to 6E may also or instead be provided at 1213B.

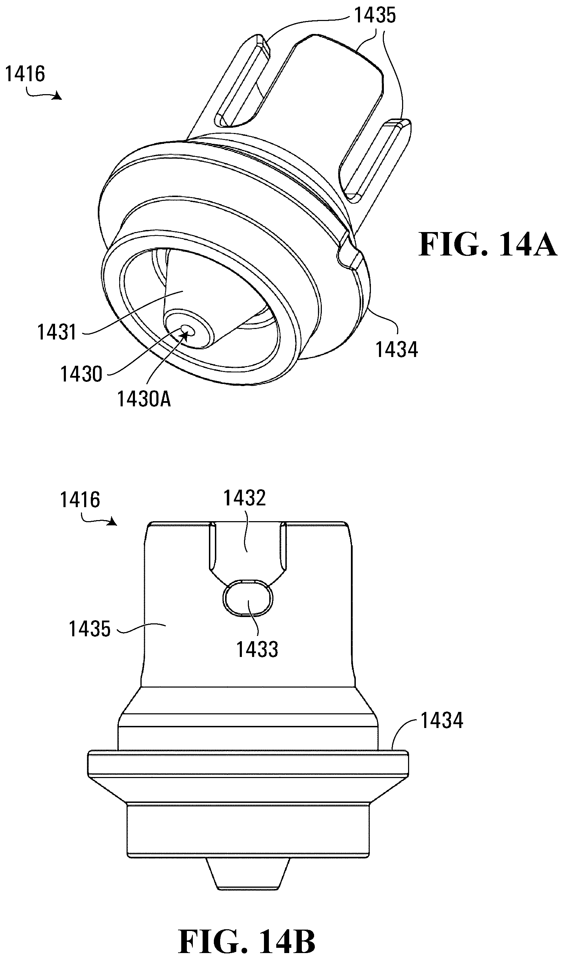

Figure 14A is a perspective view of an example adapter 1416, which may be implemented in a power tool system such as the example power tool system 1210 in Figure 12.

A hole 1430 in adapter 1416 allows a traction pin to pass through the adapter. Inner surfaces of the hole 1430 frictionally engage a traction pin in some embodiments. However, friction between the inner surfaces of the hole 1430 and a traction pin is small, so as not to prevent or substantially impede rotation of a traction pin within the hole. In some embodiments friction between inner surfaces of the hole 1430 and a traction pin is not uniform along all of the hole 1430. In some embodiments friction between inner surfaces of the hole 1430 and a traction pin is highest at an end 1430A of the hole.

A diameter of the hole 1430 is typically less than a diameter or width of a traction pin with which the adapter 1416 is intended to be used. Additionally, an inner surface of the adapter 1416 typically abuts an opposing end surface of a pin end shown in Figures 12 and 13 at 1215A when the adapter 1416 is coupled to a power tool. These features may advantageously help retain a tracking pin axially relative to the power tool.

The hole 1430 may be formed within a structure 1431 designed to support a tracking pin by preventing transverse movement of the tracking pin relative to the adapter 1416, for example. The structure 1431 preferably extends longitudinally along a portion of a tracking pin that is installed through the hole 1430. The structure 1431 may extend between 0.1 cm and 1.5 cm, for example. The hole 1430 may completely extend longitudinally through the structure 1431. In some embodiments the hole 1430 has a non-uniform diameter, as in the case of a conical structure as shown in Figure 14A, but this is not mandatory and the structure 1431 may be cylindrical, hexagonal, or another shape for example.

Inserting the adapter 1416 into the receiving end of a power tool may, for example, slide a locking pin of the receiving end of the power tool along a ramp 1432, shown by way of example in Figure 14B. Once the adapter 1416 has been sufficiently inserted, the locking pin may fall into a recess 1433, thereby locking the adapter 1416 relative to the receiving end of the power tool. In some embodiments the ramp 1432 is configured to require the adapter 1416 to be pushed into the receiving end and twisted prior to the adapter 1416 being locked relative to the receiving end of the power tool. A similar arrangement is also disclosed herein, for the pass-throughs 16, 416A, 416B for example.

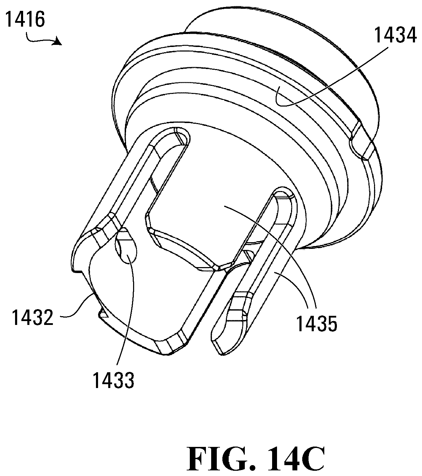

Additionally, coupling the adapter 1416 to a power tool may automatically position a surface 1434 of the adapter, shown by way of example in Figure 14C, a sufficient distance away from the power tool, or another component of a power tool system, to form a seal around an aperture in a cover.

Similar to other embodiments of a pass-through disclosed herein, the adapter 1416 in a tracking pin assembly may comprise one or more guiding protrusions 1435. Insertion of the protrusions 1435 into corresponding recesses of the receiving end of a power tool may position the adapter 1416 in a desired orientation relative to the power tool.

A tracking pin assembly 1211, with an adapter 1216, 1416, is another example of a pass-through to transmit movement from a power tool inside a cover to outside the cover. Features disclosed herein in the context of other embodiments may be implemented in a tracking pin assembly or components thereof, and similarly tracking pin assembly features disclosed herein may be implemented in other embodiments. For example, an adapter 1216, 1416 may be a multi-part device that includes first and second parts that are couplable together to trap a portion of a cover or sterile barrier and form a compression seal around an aperture, or to create an aperture and form a seal around the aperture. The end 1215A of the traction pin 1215 may be shaped or otherwise configured to couple with a power tool coupling mechanism 42 shown in Figure 4D in the context of another embodiment. An arrangement in which the movable component of a pass-through itself, such as the traction pin 1215, is or includes a tool that is to be driven by a power tool, may be implemented in other embodiments disclosed herein.

Other features and variations in a cover system and components thereof are also contemplated. As a further example, any of various sealing components or elements may be provided but have not been explicitly shown in the drawings to avoid further congestion. An O-ring, gasket, or other type of sealing component may be mounted or placed around an outer diameter of a pass-through to seal the outer diameter against a conical or cylindrical inside surface of a rigid cover or sterile barrier adapter. A sealing component may also or instead be provided as an internal O-ring, gasket, or other type of sealing component in the bore of a cover adapter and be compressed or at least seal around a cylindrical or conical pass-through. One or more sealing components may also or instead be provided inside a pass-through, to seal around one or more the bearings 572, 574 and the shaft 576 in Figure 5B for example.

Other variations may be or become apparent to those skilled in the art, based on the present disclosure.

### Interpretation of Terms

Unless the context clearly requires otherwise, throughout the description and the claims:
- "comprise", "comprising", and the like are to be construed in an inclusive sense, as opposed to an exclusive or exhaustive sense; that is to say, in the sense of "including, but not limited to";
- "connected", "coupled", or any variant thereof, means any connection or coupling, either direct or indirect, between two or more elements; the coupling or connection between the elements can be physical, logical, or a combination thereof;
- "herein", "above", "below", and words of similar import, when used to describe this specification, shall refer to this specification as a whole, and not to any particular portions of this specification;
- "or", in reference to a list of two or more items, covers all of the following interpretations of the word: any of the items in the list, all of the items in the list, and any combination of the items in the list;
- the singular forms "a", "an", and "the" also include the meaning of any appropriate plural forms.

Words that indicate directions such as "vertical", "transverse", "horizontal", "upward", "downward", "forward", "backward", "inward", "outward", "left", "right", "front", "back", "top", "bottom", "below", "above", "under", and the like, used in this description and accompanying claims, depend on the specific orientation of the apparatus described and illustrated. The subject matter described herein may assume various alternative orientations. Accordingly, these directional terms are not strictly defined and should not be interpreted narrowly.

For example, while processes or blocks such as operations involved in a method or steps of a folding sequence are presented in a given order, alternative examples may perform routines having steps, or employ systems having blocks, in a different order, and some processes or blocks may be deleted, moved, added, subdivided, combined, and/or modified to provide alternative or subcombinations. Each of these processes or blocks may be implemented in a variety of different ways. Also, while processes or blocks are at times shown as being performed in series, these processes or blocks may instead be performed in parallel, or may be performed at different times.

In addition, while elements are at times shown as being performed sequentially, they may instead be performed simultaneously or in different sequences. It is therefore intended that the following claims are interpreted to include all such variations as are within their scope.

Where a component such as an assembly, device, or part, is referred to above, unless otherwise indicated, reference to that component, including a reference to a "means" should be interpreted as including as equivalents of that component any component which performs the function of the described component and in that sense is functionally equivalent, including components which are not structurally equivalent to the disclosed structure which performs the function in the illustrated exemplary embodiments of the invention.

Specific examples of systems, methods and apparatus have been described herein for purposes of illustration. These are only examples. The technology provided herein can be applied to systems other than the example systems described above. Many alterations, modifications, additions, omissions, and permutations are possible within the practice of this invention. This invention includes variations on described embodiments that would be apparent to the skilled addressee, including variations obtained by: replacing features, elements and/or acts with equivalent features, elements and/or acts; mixing and matching of features, elements and/or acts from different embodiments; combining features, elements and/or acts from embodiments as described herein with features, elements and/or acts of other technology; and/or omitting combining features, elements and/or acts from described embodiments, as long as the variations fall within the scope of the claims.

Various features are described herein as being present in "some embodiments".

Such features, as long as they are not recited in the independent claims, are not mandatory and may not be present in all embodiments.

Embodiments of the invention may include zero, any one or any combination of two or more of such features. This is limited only to the extent that certain ones of such features are incompatible with other ones of such features in the sense that it would be impossible for a person of ordinary skill in the art to construct a practical embodiment that combines such incompatible features. Consequently, the description that "some embodiments" possess feature A and "some embodiments" possess feature B should be interpreted as an express indication that the inventors also contemplate embodiments which combine features A and B, unless the description states otherwise or features A and B are fundamentally incompatible.

## Claims

1. A disposable cover system for a power tool (12, 412, 1212), the cover system comprising:
a cover (14, 14A, 14B, 14C, 214, 414, 814, 1213) comprising a sterile outside surface, the cover defining an inner cavity (15, 415, 815), an opening (20, 820) through which the power tool is insertable into the inner cavity, and an aperture (21, 321, 521, 1213A) through which a pass-through (16, 416A, 416B, 1216) to transmit movement generated by the power tool from inside the inner cavity to outside the cover is extendable; and
a closing mechanism (24, 25) to close the opening,
the disposable cover system **characterized by** further comprising:
a compressible gasket (22, 322A, 322B, 322C, 322D, 322E, 322F, 522A, 522B, 22A, 22B, 1213B) surrounding the aperture, wherein the compressible gasket extends radially from the aperture along the sterile outside surface of the cover or an inside surface of the cover inside the inner cavity, and wherein the gasket is compressed by compression members on both sides of the cover to form a compression seal around the aperture.

2. The disposable cover system of claim 1, wherein the compressible gasket comprises a sterile compressible gasket (22, 322A, 322C, 322E, 522A, 22A, 1213B) disposed on the sterile outside surface of the cover.

3. The disposable cover system of claim 2, further comprising:
a further compressible gasket (322B, 322D, 322F, 522B, 22B), disposed on an inside surface of the cover inside the inner cavity, surrounding the aperture.

4. The disposable cover system of claim 1,
wherein the compressible gasket extends axially in the aperture,
wherein the compressible gasket further extends radially from the aperture along the sterile outside surface of the cover, radially from the aperture along an inside surface of the cover inside the inner cavity, or both radially from the aperture along the sterile outside surface of the cover and radially from the aperture along the inside surface of the cover inside the inner cavity.

5. The disposable cover system of claims 1 to 4, wherein the closing mechanism is arranged to permanently close the opening.

6. The disposable cover system of any one of claims 1 to 5, wherein the compressible gasket is compressible between compression surfaces (413, 419, 519, 590, 592, 637B, 638B, 737B, 738B, 1434) to form a compression seal around the aperture.

7. The disposable cover system of claim 6, wherein the compression surfaces comprise a first compression surface (419, 519, 1434) of the pass-through and a second compression surface (413) of the power tool.

8. The disposable cover system of 6, wherein the compression surfaces comprise a first compression surface (590) and a second compression surface (592) of the pass-through.

9. The disposable cover system of claim 6, wherein the compression surfaces comprise a first compression surface (637B, 737B) and a second compression surface (638B, 738B) on respective parts (637/737, 638/738) of a multi-part device.

10. A method comprising:
orienting an opening (20, 820) of a disposable cover (14, 14A, 14B, 14C, 214, 414, 814, 1213) to receive a power tool (12, 412, 1212) into an inner cavity (15, 415, 815) defined by the disposable cover, the disposable cover comprising a sterile outside surface and further defining an aperture (21, 321, 521, 1213A); and
operating a closing mechanism (24, 25) to close the opening with the power tool inside the inner cavity with a drive part of the power tool adjacent the aperture;
the method **characterized by** further comprising:
forming a compression seal around the aperture to seal the power tool inside the inner cavity,
wherein the forming comprises compressing one or more compressible gaskets (22, 322A, 322B, 322C, 322D, 322E, 322F, 522A, 522B, 22A, 22B, 1213B) surrounding the aperture.

11. The method of claim 10, further comprising:
placing the one or more compressible gaskets adjacent to the aperture.

12. The method of claim 10 or claim 11, wherein the forming comprises:
coupling, to the drive part of the power tool, a transmission component (16, 416A, 416B, 1216) to transmit movement from the power tool through the disposable cover, wherein the compressing comprises compressing the one or more compressible gaskets between compression surfaces (413, 419, 519, 590, 592, 637B, 638B, 737B, 738B, 1434) to form the compression seal around the aperture, wherein the compression surfaces comprise: a first compression surface (419, 519, 1434) of the transmission component and a second compression surface (413) of the power tool; or a first compression surface (590) and a second compression surface (592) of the transmission component.

13. The method of claim 10 or claim 11, wherein the forming comprises:
coupling multiple parts (637/737, 638/738) of a multi-part device together, wherein the compressing comprises compressing the one or more compressible gaskets between compression surfaces (413, 419, 519, 590, 592, 637B, 638B, 737B, 738B, 1434) to form the compression seal around the aperture, wherein the compression surfaces comprise a first compression surface (637B, 737B) and a second compression surface (638B, 738B) on respective parts of the multiple parts of the multi-part device.

14. The method of any one of claims 10 to 13, further comprising:
placing the power tool inside the inner cavity with the drive part of the power tool adjacent the aperture.

15. The method of any one of claims 10 to 14, further comprising:
creating the aperture.

## Patentansprüche

1. Ein Wegwerfabdeckungssystem für ein Elektrowerkzeug (12, 412, 1212), das Abdeckungssystem umfassend:
eine Abdeckung (14, 14A, 14B, 14C, 214, 414, 814, 1213), die eine sterile Außenfläche umfasst, wobei die Abdeckung einen inneren Hohlraum (15, 415, 815), eine Öffnung (20, 820), durch die das Elektrowerkzeug in den inneren Hohlraum einführbar ist, und eine Öffnung (21, 321, 521, 1213A) definiert, durch die ein Durchgang (16, 416A, 416B, 1216) zum Übertragen von Bewegung, die durch das Elektrowerkzeug erzeugt wird, von innerhalb des inneren Hohlraums nach außerhalb der Abdeckung ausziehbar ist; und
einen Schließmechanismus (24, 25) zum Schließen der Öffnung,
wobei das Wegwerfabdeckungssystem **dadurch gekennzeichnet ist, dass** es ferner umfasst:
eine komprimierbare Dichtung (22, 322A, 322B, 322C, 322D, 322E, 322F, 522A, 522B, 22A, 22B, 1213B), die die Öffnung umgibt, wobei sich die komprimierbare Dichtung radial von der Öffnung entlang der sterilen Außenfläche der Abdeckung oder einer Innenfläche der Abdeckung innerhalb des inneren Hohlraums erstreckt, und
wobei die Dichtung durch Kompressionselemente auf beiden Seiten der Abdeckung komprimiert wird, um eine Kompressionsdichtung um die Öffnung zu bilden.

2. Das Wegwerfabdeckungssystem nach Anspruch 1, wobei die komprimierbare Dichtung eine sterile komprimierbare Dichtung (22, 322A, 322C, 322E, 522A, 22A, 1213B) umfasst, die auf der sterilen Außenfläche der Abdeckung angeordnet ist.

3. Das Wegwerfabdeckungssystem nach Anspruch 2, ferner umfassend:
eine weitere komprimierbare Dichtung (322B, 322D, 322F, 522B, 22B), die auf einer Innenfläche der Abdeckung innerhalb des inneren Hohlraums angeordnet ist und die Öffnung umgibt.

4. Das Wegwerfabdeckungssystem nach Anspruch 1,
wobei sich die komprimierbare Dichtung axial in der Öffnung erstreckt,
wobei sich die komprimierbare Dichtung ferner radial von der Öffnung entlang der sterilen Außenfläche der Abdeckung, radial von der Öffnung entlang einer Innenfläche der Abdeckung innerhalb des inneren Hohlraums oder sowohl radial von der Öffnung entlang der sterilen Außenfläche der Abdeckung als auch radial von der Öffnung entlang der Innenfläche der Abdeckung innerhalb des inneren Hohlraums erstreckt.

5. Das Wegwerfabdeckungssystem nach den Ansprüchen 1 bis 4, wobei der Schließmechanismus angeordnet ist, um die Öffnung dauerhaft zu schließen.

6. Das Wegwerfabdeckungssystem nach einem der Ansprüche 1 bis 5, wobei die komprimierbare Dichtung zwischen Kompressionsflächen (413, 419, 519, 590, 592, 637B, 638B, 737B, 738B, 1434) komprimierbar ist, um eine Kompressionsdichtung um die Öffnung zu bilden.

7. Das Wegwerfabdeckungssystem nach Anspruch 6, wobei die Kompressionsflächen eine erste Kompressionsfläche (419, 519, 1434) des Durchgangs und eine zweite Kompressionsfläche (413) des Elektrowerkzeugs umfassen.

8. Das Wegwerfabdeckungssystem nach Anspruch 6, wobei die Kompressionsflächen eine erste Kompressionsfläche (590) und eine zweite Kompressionsfläche (592) des Durchgangs umfassen.

9. Das Wegwerfabdeckungssystem nach Anspruch 6, wobei die Kompressionsflächen eine erste Kompressionsfläche (637B, 737B) und eine zweite Kompressionsfläche (638B, 738B) an jeweiligen Teilen (637/737, 638/738) einer mehrteiligen Vorrichtung umfassen.

10. Ein Verfahren, umfassend:
Ausrichten einer Öffnung (20, 820) einer Wegwerfabdeckung (14, 14A, 14B, 14C, 214, 414, 814, 1213), um ein Elektrowerkzeug (12, 412, 1212) in einem inneren Hohlraum (15, 415, 815), der durch die Wegwerfabdeckung definiert ist, aufzunehmen, wobei die Wegwerfabdeckung eine sterile Außenfläche umfasst und ferner eine Öffnung (21, 321, 521, 1213A) definiert; und
Betreiben eines Schließmechanismus (24, 25), um die Öffnung mit dem Elektrowerkzeug innerhalb des inneren Hohlraums mit einem Antriebsteil des Elektrowerkzeugs angrenzend an die Öffnung zu schließen;
wobei das Verfahren **dadurch gekennzeichnet ist, dass** es ferner umfasst:
Bilden einer Kompressionsdichtung um die Öffnung, um das Elektrowerkzeug innerhalb des inneren Hohlraums abzudichten,
wobei das Bilden das Komprimieren einer oder mehrerer komprimierbarer Dichtungen (22, 322A, 322B, 322C, 322D, 322E, 322F, 522A, 522B, 22A, 22B, 1213B), die die Öffnung umgeben, umfasst.

11. Das Verfahren nach Anspruch 10, ferner umfassend:
Platzieren der einen oder mehreren komprimierbaren Dichtungen angrenzend an die Öffnung.

12. Das Verfahren nach Anspruch 10 oder Anspruch 11, wobei das Bilden umfasst:
Koppeln, an das Antriebsteil des Elektrowerkzeugs, einer Getriebekomponente (16, 416A, 416B, 1216), um Bewegung von dem Elektrowerkzeug durch die Wegwerfabdeckung zu übertragen, wobei das Komprimieren Komprimieren der einen oder mehreren komprimierbaren Dichtungen zwischen Kompressionsflächen (413, 419, 519, 590, 592, 637B, 638B, 737B, 738B, 1434) umfasst, um die Kompressionsdichtung um die Öffnung zu bilden, wobei die Kompressionsflächen umfassen: eine erste Kompressionsfläche (419, 519, 1434) der Getriebekomponente und eine zweite Kompressionsfläche (413) des Elektrowerkzeugs; oder eine erste Kompressionsfläche (590) und eine zweite Kompressionsfläche (592) der Getriebekomponente.

13. Das Verfahren nach Anspruch 10 oder Anspruch 11, wobei das Bilden umfasst:
Koppeln mehrerer Teile (637/737, 638/738) einer mehrteiligen Vorrichtung miteinander, wobei das Komprimieren Komprimieren der einen oder mehreren komprimierbaren Dichtungen zwischen Kompressionsflächen (413, 419, 519, 590, 592, 637B, 638B, 737B, 738B, 1434) umfasst, um die Kompressionsdichtung um die Öffnung zu bilden, wobei die Kompressionsflächen eine erste Kompressionsfläche (637B, 737B) und eine zweite Kompressionsfläche (638B, 738B) an jeweiligen Teilen der mehreren Teile der mehrteiligen Vorrichtung umfassen.

14. Das Verfahren nach einem der Ansprüche 10 bis 13, ferner umfassend:
Platzieren des Elektrowerkzeugs innerhalb des inneren Hohlraums mit dem Antriebsteil des Elektrowerkzeugs angrenzend an die Öffnung.

15. Das Verfahren nach einem der Ansprüche 10 bis 14, ferner umfassend:
Erzeugen der Öffnung.

## Revendications

1. Un système de revêtement jetable pour un outil électrique (12, 412, 1212), le système de revêtement comprenant :
un revêtement (14, 14A, 14B, 14C, 214, 414, 814, 1213) comprenant une surface extérieure stérile, le revêtement définissant une cavité interne (15, 415, 815), une ouverture (20, 820) par laquelle l'outil électrique est apte à être inséré dans la cavité interne, et un orifice (21, 321, 521, 1213A) par lequel un passage traversant (16, 416A, 416B, 1216), pour transmettre un mouvement généré par l'outil électrique de l'intérieur de la cavité interne vers l'extérieur du revêtement, est extensible ; et
un mécanisme de fermeture (24, 25) pour fermer l'ouverture,
le système de revêtement jetable étant **caractérisé par le fait qu'**il comprend en outre :
un joint compressible (22, 322A, 322B, 322C, 322D, 322E, 322F, 522A, 522B, 22A, 22B, 1213B) entourant l'orifice, dans lequel le joint compressible s'étend radialement à partir de l'orifice le long de la surface extérieure stérile du revêtement ou d'une surface intérieure du revêtement à l'intérieur de la cavité interne, et dans lequel le joint est comprimé par des éléments de compression sur les deux côtés du revêtement pour former un joint de compression autour de l'orifice.

2. Le système de revêtement jetable selon la revendication 1, dans lequel le joint compressible comprend un joint compressible stérile (22, 322A, 322C, 322E, 522A, 22A, 1213B) disposé sur la surface extérieure stérile du revêtement.

3. Le système de revêtement jetable selon la revendication 2, comprenant en outre :
un joint compressible supplémentaire (322B, 322D, 322F, 522B, 22B), disposé sur une surface intérieure du revêtement à l'intérieur de la cavité interne, entourant l'orifice.

4. Le système de revêtement jetable selon la revendication 1,
dans lequel le joint compressible s'étend axialement dans l'orifice,
dans lequel le joint compressible s'étend en outre radialement à partir de l'orifice le long de la surface extérieure stérile du revêtement, radialement à partir de l'orifice le long d'une surface intérieure du revêtement à l'intérieur de la cavité interne, ou à la fois radialement à partir de l'orifice le long de la surface extérieure stérile du revêtement et radialement à partir de l'orifice le long de la surface intérieure du revêtement à l'intérieur de la cavité interne.

5. Le système de revêtement jetable selon les revendications 1 à 4, dans lequel le mécanisme de fermeture est agencé pour fermer de manière permanente l'ouverture.

6. Le système de revêtement jetable selon l'une des revendications 1 à 5, dans lequel le joint compressible est compressible entre des surfaces de compression (413, 419, 519, 590, 592, 637B, 638B, 737B, 738B, 1434) pour former un joint de compression autour de l'orifice.

7. Le système de revêtement jetable selon la revendication 6, dans lequel les surfaces de compression comprennent une première surface de compression (419, 519, 1434) du passage traversant et une deuxième surface de compression (413) de l'outil électrique.

8. Le système de revêtement jetable selon la revendication 6, dans lequel les surfaces de compression comprennent une première surface de compression (590) et une deuxième surface de compression (592) du passage traversant.

9. Le système de revêtement jetable selon la revendication 6, dans lequel les surfaces de compression comprennent une première surface de compression (637B, 737B) et une deuxième surface de compression (638B, 738B) sur des parties (637/737, 638/738) respectives d'un dispositif en plusieurs parties.

10. Un procédé comprenant :
l'orientation d'une ouverture (20, 820) d'un revêtement jetable (14, 14A, 14B, 14C, 214, 414, 814, 1213) pour recevoir un outil électrique (12, 412, 1212) dans une cavité interne (15, 415, 815) définie par le revêtement jetable, le revêtement jetable comprenant une surface extérieure stérile et définissant en outre un orifice (21, 321, 521, 1213A) ; et
la mise en œuvre d'un mécanisme de fermeture (24, 25) pour fermer l'ouverture avec l'outil électrique à l'intérieur de la cavité interne avec une partie d'entraînement de l'outil électrique adjacente à l'orifice ;
le procédé étant **caractérisé par le fait qu'**il comprend en outre :
la formation d'un joint de compression autour de l'orifice pour rendre étanche l'outil électrique à l'intérieur de la cavité interne,
dans lequel la formation comprend la compression d'un ou de plusieurs joints compressibles (22, 322A, 322B, 322C, 322D, 322E, 322F, 522A, 522B, 22A, 22B, 1213B) entourant l'orifice.

11. Le procédé selon la revendication 10, comprenant en outre :
le placement d'un ou de plusieurs joints compressibles de manière adjacente à l'orifice.

12. Le procédé selon la revendication 10 ou la revendication 11, dans lequel la formation comprend :
le couplage, avec la partie d'entraînement de l'outil électrique, d'un composant de transmission (16, 416A, 416B, 1216) pour transmettre un mouvement de l'outil électrique à travers le revêtement jetable, dans lequel la compression comprend la compression des un ou plusieurs joints compressibles entre des surfaces de compression (413, 419, 519, 590, 592, 637B, 638B, 737B, 738B, 1434) pour former le joint de compression autour de l'orifice, dans lequel les surfaces de compression comprennent : une première surface de compression (419, 519, 1434) du composant de transmission et une deuxième surface de compression (413) de l'outil électrique ; ou une première surface de compression (590) et une deuxième surface de compression (592) du composant de transmission.

13. Le procédé selon la revendication 10 ou la revendication 11, dans lequel la formation comprend :
le couplage de plusieurs parties (637/737, 638/738) d'un dispositif en plusieurs parties, dans lequel la compression comprend la compression des un ou plusieurs joints compressibles entre des surfaces de compression (413, 419, 519, 590, 592, 637B, 638B, 737B, 738B, 1434) pour former le joint de compression autour de l'orifice, dans lequel les surfaces de compression comprennent une première surface de compression (637B, 737B) et une deuxième surface de compression (638B, 738B) sur des parties respectives des multiples parties du dispositif en plusieurs parties.

14. Le procédé selon l'une des revendications 10 à 13, comprenant en outre :
le placement de l'outil électrique à l'intérieur de la cavité interne avec la partie d'entraînement de l'outil électrique adjacente à l'orifice.

15. Le procédé selon l'une des revendications 10 à 14, comprenant en outre :
la création de l'orifice.
